(19) Europäisches Patentamt
European Patent Office
Office européen des brevets

(11) Veröffentlichungsnummer: **0 014 951**
**B1**

(12) # EUROPÄISCHE PATENTSCHRIFT

(45) Veröffentlichungstag der Patentschrift: **12.01.83**

(21) Anmeldenummer: **80100719.6**

(22) Anmeldetag: **13.02.80**

(51) Int. Cl.³: **A 61 K 31/395,** C 07 D 209/08, C 07 D 209/42, C 07 D 249/18, C 07 D 235/08, C 07 D 235/26, C 07 D 235/28, C 07 D 235/02, C 07 D 231/56, C 07 D 405/12, C 07 D 401/12

(54) **Heterocyclische Oxypropanolamin-Derivate, Verfahren zu ihrer Herstellung und diese Verbindungen enthaltende Arzneimittel.**

(30) Priorität: **16.02.79 DE 2905877**

(43) Veröffentlichungstag der Anmeldung: **03.09.80 Patentblatt 80/18**

(45) Bekanntmachung des Hinweises auf die Patenterteilung: **12.01.83 Patentblatt 83/2**

(84) Benannte Vertragsstaaten: **AT BE CH DE FR GB IT LU NL SE**

(56) Entgegenhaltungen:
**EP-A-0 003 758**
**CH-A- 483 423**
**CH-A- 536 839**
**DE-A-2 113 397**
**DE-A-2 113 733**
**DE-A-2 230 426**
**DE-A-2 700 193**
**DE-A-2 830 211**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Friebe, Walter-Gunar, Dr. rer. nat., Oberstrasse 13, D-6100 Darmstadt (DE)**
Erfinder: **Michel, Helmut, Ziegelgasse 2a, D-6800 Mannheim 31 (DE)**
Erfinder: **Ross, Carl Heinz, Dr. rer. nat., Rathausstrasse 45, D-6806 Viernheim (DE)**
Erfinder: **Wiedemann, Fritz, Dr. phil., Weinheimer Strasse 82, D-6940 Weinheim-Lützelsachsen (DE)**
Erfinder: **Bartsch, Wolfgang, Dr. med. vet., Franconviller-Strasse 5, D-6806 Viernheim (DE)**
Erfinder: **Dietmann, Karl, Prof. Dr. med., Eisenacher Weg 75, D-6800 Mannheim-Vogelstang (DE)**

**EP 0 014 951 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Heterocyclische Oxypropanolamin-Derivate, Verfahren zu ihrer Herstellung und diese Verbindungen enthaltende Arzneimittel

Die vorliegende Erfindung betrifft neue heterocyclische Oxypropanolamin-Derivate der allgemeinen Formel I

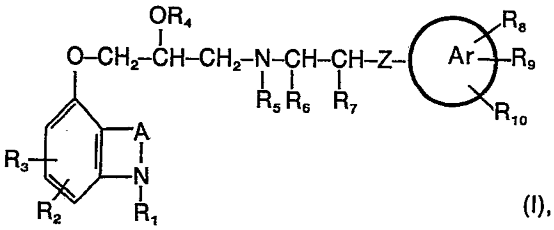

(I),

in welcher

$R_1$ Wasserstoff, eine niedere Alkyl-, Aralkyl- oder niedere Alkanoylgruppe,

$R_2$ und $R_3$, die gleich oder verschieden sein können, jeweils Wasserstoff, eine niedere Alkyl-, Hydroxyalkyl-, Alkoxycarbonyl- oder Alkanoyloxyalkylgruppe sowie gemeinsam einen Alkylenrest,

$R_4$ Wasserstoff, eine niedere Alkanoylgruppe oder eine Aroylgruppe,

$R_5$ Wasserstoff, eine niedere Alkylgruppe oder eine Aralkylgruppe,

$R_6$ Wasserstoff oder eine niedere Alkylgruppe,

$R_7$ Wasserstoff, eine Hydroxygruppe oder eine niedere Alkylgruppe,

Z einen Valenzstrich, eine Methylengruppe, ein Sauerstoff- oder Schwefelatom,

Ar einen carbocyclischen Arylrest oder einen Pyridylrest,

$R_8$, $R_9$ und $R_{10}$, die gleich oder verschieden sein können, jeweils Wasserstoff, Halogen, eine Hydroxygruppe, eine niedere Alkanoylgruppe, eine niedere Alkylgruppe, eine niedere Alkenylgruppe, eine niedere Alkoxygruppe, eine Aralkoxygruppe, die Allyloxygruppe, eine niedere Alkylthiogruppe, eine Aminocarbonylgruppe, eine Aminosulfonylgruppe, eine niedere Alkanoylaminogruppe oder $R_8$ und $R_9$ gemeinsam eine Niederalkylendioxygruppe oder $R_8$ gemeinsam mit $R_7$ auch eine $-CH_2-O-$Gruppe und

A die Gruppe $X_1-Y_1$, in der $X_1$ einen Methylen- oder $-NR_{11}$-Rest, wobei $R_{11}$ Wasserstoff oder eine niedere Alkylgruppe bedeutet, und $Y_1$ einen Methylenrest oder die Gruppe $>C=Q$, wobei Q für Sauerstoff oder Schwefel steht, vorstellen, oder die Gruppe $X_2=Y_2$, in der $X_2$ und $Y_2$ gleich oder verschieden sein können und jeweils für ein Stickstoffatom oder für eine $=\overset{|}{C}R_{12}$-Gruppe stehen, wobei $R_{12}$ Wasserstoff, eine niedere Alkyl- oder Alkoxycarbonylgruppe vorstellt, wobei für den Fall, dass $X_2=Y_2$ eine $-CH=N$-Gruppe und $R_1$ eine Alkyl- oder Aralkylgruppe vorstellen, diese wegen der Tautomeriefähigkeit der Indazole auch an dem für $Y_2$ stehenden Stickstoffatom lokalisiert sein kann, mit der Massgabe, dass jeweils $Y_1$ mit dem Rest $N-R_1$ der allgemeinen Formel I verbunden ist, bedeuten, wobei für den Fall, dass Q ein Sauerstoffatom oder $X_2=Y_2$ eine $-CR_{12}=CR_{12}$-Gruppe bedeutet, entweder die beiden Reste $R_2$ und $R_3$ nicht gleichzeitig Wasserstoff sein können oder $R_7$ und $R_8$ zusammen eine $-CH_2-O-$Brücke bilden müssen, sowie deren pharmakologisch verträglichen Salze.

Da die Verbindungen der Formel I asymmetrische Kohlenstoffatome besitzen, sind ferner Gegenstand der Erfindung die optisch aktiven Formen und racemische Gemische dieser Verbindungen.

Die Verbindungen der Formel I und ihre pharmakologisch verträglichen Salze besitzen bei geringer Toxizität ausgeprägte vasodilatierende Eigenschaften, die sich im wesentlichen in einer Senkung des Blutdrucks äussern; ausserdem wird eine Hemmung adrenergischer β-Rezeptoren beobachtet. Die erfindungsgemässen Verbindungen eignen sich daher besonders zur Behandlung und Prophylaxe bei Herz- und Kreislauferkrankungen.

In den deutschen Offenlegungsschriften 1 948 507, 2 113 379, 2 230 426, 2 619 164, 2 651 574, 2 700 193 und 2 830 211 sowie in der Schweizer Patentschrift 483 423 sind Verbindungen ähnlicher Struktur und Wirkung beschrieben und beansprucht. Durch Abänderung des heterocyclischen Phenolteils sowie der Aminopropoxy-Seitenkette wurde eine überraschende Wirkungsverbesserung erzielt.

Unter einer niederen Alkylgruppe der Substituenten $R_1$, $R_2$, $R_3$, $R_5$, $R_6$, $R_7$, $R_8$, $R_9$, $R_{10}$, $R_{11}$ und $R_{12}$ sind geradkettige oder verzweigte Gruppen mit 1–6, vorzugsweise 1–4 Kohlenstoffatomen zu verstehen, wie z.B. Methyl, Ethyl, Propyl, Isopropyl, Butyl, Isobutyl, tert.-Butyl oder n-Hexyl. Insbesondere kommt jedoch die Methyl- oder Ethylgruppe in Frage. Der von den Substituenten $R_2$ und $R_3$ gegebenenfalls gemeinsam gebildete Alkylenrest weist 3–4, vorzugsweise 3 Kohlenstoffatome auf.

Unter Alkenylgruppe sind ungesättigte Kohlenwasserstoffreste mit bis 6 C-Atomen zu verstehen, insbesondere der Allyl- und Methylvinyl-Rest.

Hydroxyalkylgruppen der Substituenten $R_2$ und $R_3$ enthalten 1–4 Kohlenstoffatome, bevorzugt ist die 2-Hydroxyethyl- und die Hydroxymethylgruppe.

Alkoxygruppen der Substituenten $R_8$, $R_9$ und $R_{10}$ enthalten 1–6, vorzugsweise 1–4 Kohlenstoffatome, wie z.B. die Methoxy-, Ethoxy-, Propoxy-, Butoxy- oder Pentoxygruppe. Bevorzugt sind die Methoxy-, Ethoxy- und Propoxygruppe.

Als Alkoxycarbonylgruppe der Substituenten $R_2$, $R_3$ und $R_{12}$ kommen insbesondere die Methoxycarbonyl- und Ethoxycarbonylgruppe in Frage.

Alkylthiogruppen der Substituenten $R_8$, $R_9$ und $R_{10}$ sind Gruppen mit 1–6, vorzugsweise 1–4 Kohlenstoffatomen. Bevorzugt ist der Methylmercaptorest.

Alkanoylgruppen der Substituenten $R_1$, $R_4$, $R_8$, $R_9$ und $R_{10}$ sowie der Alkanoylteil der Alkanoyloxyalkylgruppe in der Definition der Substituenten $R_2$

und $R_3$ und der Alkanoylaminogruppe der Substituenten $R_8$, $R_9$ und $R_{10}$ enthalten 1–8, vorzugsweise 1–5 Kohlenstoffatome, wobei deren Alkylgruppen geradkettig oder verzweigt sein können. Bevorzugt sind der Formyl-, Acetyl- oder der Pivaloylrest. Als Alkanoyloxyalkylgruppen sind Alkanoyloxymethylgruppen besonders bevorzugt.

Unter einer Aroylgruppe in der Definition des Substituenten $R_4$ ist bevorzugt die Benzoylgruppe zu verstehen, die ein- oder mehrfach durch Halogen, Alkylgruppen mit 1–4 Kohlenstoffatomen oder Alkoxygruppen mit 1–4 Kohlenstoffatomen substituiert sein kann.

Unter einer Aralkylgruppe in der Definition der Substituenten $R_1$ und $R_5$ sowie unter einer Aralkoxygruppe in der Definition der Substituenten $R_8$, $R_9$ und $R_{10}$ sind Reste zu verstehen, die als Arylteil einen Phenyl- oder Naphthylrest und als Alkylteil eine gerade oder verzweigte gesättigte Kohlenwasserstoffkette mit 1–4 Kohlenstoffatomen aufweisen. Besonders bevorzugt ist der Benzylrest.

Der carbocyclische Arylrest Ar kann ein Phenyl- oder auch Naphthylrest sein, wobei der Phenylrest besonders bevorzugt ist.

Als von den Substituenten $R_8$ und $R_9$ gemeinsam gebildete Niederalkylendioxygruppe kommen vorzugsweise die Methylen- und Ethylendioxygruppe in Frage.

Das Zeichen A in der allgemeinen Formel I hat insbesondere die Bedeutung, dass als Heterocyclen Benzimidazolinon-2, Benzimidazolinthion-2, Benzimidazol, Benztriazol, Indol, Indolin und Indazol zu verstehen sind.

Unter Halogen wird im Sinne der Erfindung Fluor, Chlor, Brom und Jod verstanden, insbesondere Fluor, Chlor und Brom.

Die Herstellung der neuen Verbindungen der allgemeinen Formel I ist dadurch gekennzeichnet, dass man in an sich bekannter Weise entweder

a) eine Verbindung der Formel II

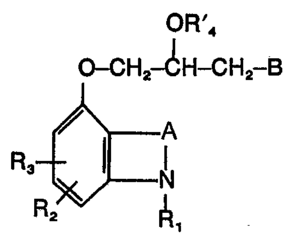

(II),

in welcher $R_1$, $R_2$, $R_3$ und A die oben genannte Bedeutung haben, B eine reaktive Gruppe darstellt und $R_4'$ die oben angegebene Bedeutung für $R_4$ hat oder zusammen mit B einen Valenzstrich bedeutet, mit einer Verbindung der Formel III

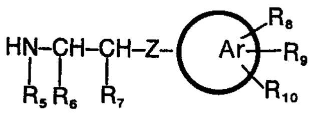

(III),

in welcher $R_5$, $R_6$, $R_7$, $R_8$, $R_9$, $R_{10}$, Z und Ar die oben angegebene Bedeutung haben, umsetzt oder

b) eine Verbindung der Formel IV

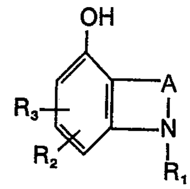

(IV),

in welcher $R_1$, $R_2$, $R_3$ und A die angegebene Bedeutung haben, mit einer Verbindung der Formel V

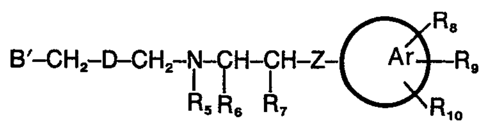

(V),

in welcher $R_6$, $R_7$, $R_8$, $R_9$, $R_{10}$, Z und Ar die oben angegebene Bedeutung haben, B′ eine reaktive Gruppe darstellt, D für eine >CO- oder >CH–$OR_4''$-Gruppe steht, wobei $R_4''$ die oben genannte Bedeutung für $R_4$ hat oder auch zusammen mit B′ einen Valenzstrich bedeutet und $R_5'$ die oben genannte Bedeutung für $R_5$ hat oder auch zusammen mit B′ eine Einfachbindung sein kann, umsetzt und, falls D die >CO-Gruppe bedeutet, anschliessend reduziert oder

c) eine Verbindung der Formel VI

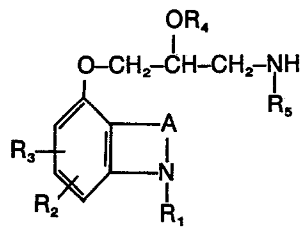

(VI),

in welcher $R_1$, $R_2$, $R_3$, $R_4$, $R_5$ und A die oben angegebene Bedeutung haben, mit einer Verbindung der Formel VII

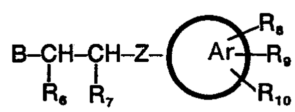

(VII),

in welcher B, $R_6$, $R_7$, $R_8$, $R_9$, $R_{10}$, Z und Ar die angegebene Bedeutung haben, umsetzt oder

d) ein Gemisch aus einer Verbindung der Formel VI mit einer Verbindung der Formel VIII

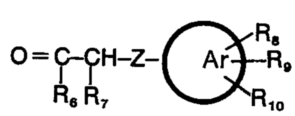

(VIII),

in welcher $R_6$, $R_7$, $R_8$, $R_9$, $R_{10}$, Z und Ar die angegebene Bedeutung haben, hydriert oder

e) eine Verbindung der Formel VI mit einer Verbindung der Formel IX

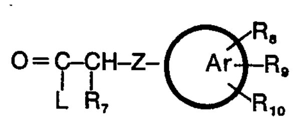

(IX),

in welcher $R_7$, $R_8$, $R_9$, $R_{10}$, Z und Ar die angegebene Bedeutung haben und L einen reaktiven Rest darstellt, umsetzt und das erhaltene Amid anschliessend reduziert oder

f) eine Verbindung der Formel X

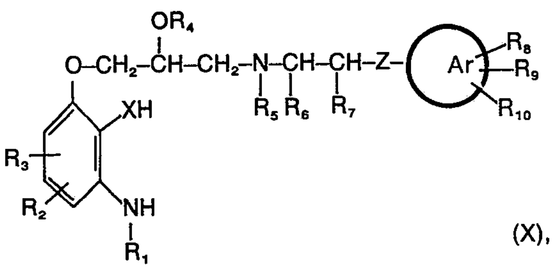

(X),

in welcher $R_1$, $R_2$, $R_3$, $R_4$, $R_5$, $R_6$, $R_7$, $R_8$, $R_9$, $R_{10}$, Z und Ar die angegebene Bedeutung haben und $XX_1$ oder $HX_2$ vorstellt, für den Fall, dass A die Gruppe $X_1$–$Y_1$ bedeutet, eine Verbindung, in der X = $X_1$ darstellt, mit einer Verbindung der allgemeinen Formel XIa

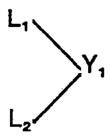

(XIa)

in der $Y_1$ die oben angegebene Bedeutung hat, $L_1$ ein Wasserstoffatom, die Hydroxygruppe oder einen reaktiven Rest T und $L_2$ ein Wasserstoffatom oder einen reaktiven Rest T bedeuten, oder für den Fall, dass die Gruppe $X_2$=$Y_2$ bedeutet, eine Verbindung, in der X = $HX_2$ darstellt, mit einer Verbindung der allgemeinen Formel XIb

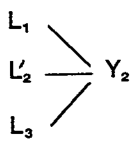

(XIb),

in der $L_1$ und $Y_2$ die oben angegebene Bedeutung haben, $L_2'$ ein Wasserstoffatom oder einen reaktiven Rest T und $L_3$ ein Wasserstoffatom oder zusammen mit $L_2'$ ein Sauerstoffatom darstellt, umsetzt und cyclisiert oder

g) eine Verbindung der Formel XII

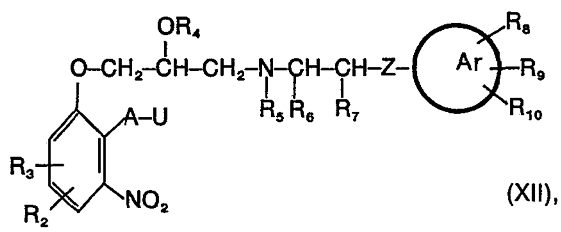

(XII),

in welcher $R_2$, $R_3$, $R_4$, $R_5$, $R_6$, $R_7$, $R_8$, $R_9$, $R_{10}$, Z, A und Ar die angegebene Bedeutung haben und U eine abspaltbare Gruppe darstellt, reduziert und cyclisiert und gegebenenfalls nachträglich in einer erhaltenen Verbindung der allgemeinen Formel I einen der Reste $R_1$, $R_2$, $R_3$, $R_4$, $R_5$, $R_8$, $R_9$, $R_{10}$ oder $R_{12}$ nach üblichen Methoden in einen anderen, durch den Anspruch definierten Rest $R_1$, $R_2$, $R_3$, $R_4$, $R_5$, $R_8$, $R_9$, $R_{10}$ oder $R_{12}$ überführt und die erhaltenen Verbindungen gewünschtenfalls in ihre pharmakologisch verträglichen Salze umwandelt.

Reaktive Reste B in Verbindungen der allgemeinen Formeln II und VII sowie B' in Verbindungen der allgemeinen Formel V sind insbesondere Säurereste, z.B. von Halogenwasserstoffsäuren und Sulfonsäuren. Besonders bevorzugt sind Chloride, Mesyloxy- und Tosyloxyreste.

Reaktive Reste L in Verbindungen der allgemeinen Formel IX sind insbesondere Säurereste, z.B. von Halogenwasserstoffsäuren oder Stickstoffwasserstoffsäure oderr von Niederalkancarbonsäuren, Niederalkoxy- oder Aryloxygruppen, Amino- oder Imidazolylgruppen. Besonders bevorzugt sind Chloride, Azide und Imidazolylreste.

Die reaktiven Reste T und U in Verbindungen der Formeln XIa, XIb und XII stehen für alle Reste, die sich nuclophil substituieren lassen. Solche Reste sind vorzugsweise Halogenatome, wie Chlor oder Brom, Amino-, Imidazolyl-, Niederalkoxy-, Niederacyloxy-, Phenoxygruppen, Mercapto- und Niederalkoxythiocarbonylgruppen. Beispielsweise können als Verbindungen der allgemeinen Formel XIa Carbonylhalogenide, Harnstoff, N,N'-Carbonyldiimidazol, sowie für den Fall, dass Q für Schwefel steht, Thiocarbonylhalogenide, Thioharnstoff oder auch Xanthogenate eingesetzt werden. Verbindungen der allgemeinen Formel XIa können auch in situ aus anderen Verbindungen, z.B. Schwefelkohlenstoff in alkalischer Lösung, in der Reaktionslösung hergestellt werden. Als Verbindungen der allgemeinen Formel XIb kommen beispielsweise Carbonsäuren, wie Ameisensäure oder Essigsäure, Carbonsäureester oder auch Carbonsäurehalogenide in Frage. Verbindungen XIb können jedoch auch in situ aus anderen Substanzen in der Reaktionsmischung hergestellt werden, z.B. anorganisches Nitrit in wässriger Mineralsäure, Niederalkylsalpetrigsäureester in organischen Lösungsmitteln.

Die erfindungsgemässen Verfahren werden zweckmässig in einem unter den Reaktionsbedingungen inerten Lösungsmittel, z.B. Wasser, Methanol, Ethanol, n-Butanol, Dioxan, Dimethylformamid oder Hexamethylphosphorsäuretriamid, gegebenenfalls in Gegenwart eines säurebindenden Mittels durchgeführt. Die Umsetzungen können auch durch Mischen der Reaktionskomponenten ohne Lösungsmittel erreicht werden. Die Reaktionen werden bei Raumtemperatur oder unter Erwärmen, gegebenenfalls unter einer Schutzgasatmosphäre ausgeführt.

Die gegebenenfalls durchzuführende Reduktion der für D stehenden Gruppe >C=O erfolgt zweckmässig durch katalytische Hydrierung mit Edelmetall- oder Nickelkatalysatoren oder mittels kompleter Metallhydride, wie z.B. Natriumborhydrid.

Die Hydrierung eines Amins der Formel VI mit einer Carbonylverbindung der Formel VIII gemäss Verfahren d) wird in einem geeigneten Lösungsmittel (z.B. Methanol) in Gegenwart eines Katalysators (z.B. Raney-Nickel) durchgeführt.

Die Reduktion der nach Verfahren e) erhaltenen Amide erfolgt vorzugsweise mittels komplexer Metallhydride, z.B. Lithiumaluminiumhydrid.

Die bei den erfindungsgemässen Verfahren eingesetzten Ausgangsverbindungen sind in der Regel literaturbekannte Verbindungen. Neue Verbindungen werden im allgemeinen analog der für die Herstellung dieser bekannten Verbindungen beschriebenen Verfahren erhalten. So können beispielsweise die Amine der allgemeinen Formel III durch Umsetzung von Halogen-alkyl-nitrilen mit entsprechenden Phenolen, Naphtholen oder Aryl-Verbindungen (z.B. Chloracetonitril und Phenol) und anschliessender Hydrierung in Gegenwart von Ammoniak hergestellt werden.

Die Amine der allgemeinen Formel VI können aus den entsprechenden (2,3-Epoxypropoxy)-Derivaten durch Umsetzung mit flüssigem Ammoniak erhalten werden.

Reaktive Verbindungen der allgemeinen Formel VII, z.B. p-Toluolsulfonsäureester werden in der Regel aus den entsprechenden Phenolen, Naphtholen, Aryl- oder Pyridyl-Verbindungen durch Umsetzung mit Halogenalkoholen und anschliessender Veresterung mit p-Toluolsulfonsäure hergestellt.

Die Carbonyl-Verbindungen der allgemeinen Formel VIII und die reaktiven Säurederivate der allgemeinen Formel IX werden z.B. aus entsprechenden Phenolen, Naphtholen, Aryl- und Pyridylverbindungen durch Umsetzen mit geeigneten Halogenalkylverbindungen erhalten.

Als gegebenenfalls nachträglich durchzuführende Umwandlung eines der Substituenten $R_1$, $R_2$, $R_3$, $R_4$, $R_5$, $R_8$, $R_9$, $R_{10}$ oder $R_{12}$ in Verbindungen der allgemeinen Formel I in einen anderen, durch den Anspruch definierten Rest $R_1$, $R_2$, $R_3$, $R_4$, $R_5$, $R_8$, $R_9$, $R_{10}$ oder $R_{12}$ kommen beispielsweise die nachträgliche Acylierung einer OH–Gruppe zu einer Alkanoyloxy- bzw. Aroyloxy-Gruppe,

die Reduktion einer Alkoxycarbonylgruppe zu einem Hydroxymethylrest,

die Hydrolyse einer Alkanoyloxymethylgruppe zu einem Hydroxymethylrest,

die Reduktion einer Alkanoyloxymethylgruppe zu einem Methylsubstituenten oder

die Abspaltung einer Benzylgruppe in Frage.

Die Veresterung einer für $-OR_4$ stehenden Hydroxygruppe kann in an sich üblicher Weise durch Umsetzung mit einem Säurehalogenid oder Säureanhydrid, gegebenenfalls in Gegenwart eines säurebindenden Mittels, wie z.B. Pyridin oder Triethylamin erfolgen.

Die gegebenenfalls durchzuführende Reduktion von Verbindungen der allgemeinen Formel I, in denen $R_2$ und/oder $R_3$ eine Alkoxycarbonyl- bzw. eine Alkanoyloxymethylgruppe vorstellen, in Verbindungen der Formel I, in denen $R_2$ und/oder $R_3$ einen Hydroxymethyl- bzw. Methyl-Substituenten darstellen, erfolgt zweckmässig mittels komplexer Metallhydride, wie z.B. Lithiumaluminiumhydrid oder durch katalytische Hydrierung mittels Edelmetallkatalysatoren oder Raney-Nickel.

Die Hydrolyse von Alkanoyloxymethylgruppen $R_2$ und/oder $R_3$ in Verbindungen der allgemeinen Formel I zu Hydroxymethylresten $R_2$ und/oder $R_3$ kann in an sich bekannter Weise sauer oder alkalisch erfolgen.

Die Abspaltung einer für $R_1$ und $R_5$ stehenden bzw. in $R_8$, $R_9$ oder $R_{10}$ enthaltenen Benzylgruppe erfolgt beispielsweise durch Hydrierung in Gegenwart von Edelmetallkatalysatoren.

Zur Überführung der Verbindungen der Formel I in ihre pharmakologisch unbedenklichen Salze setzt man diese vorzugsweise in einem organischen Lösungsmittel mit einer anorganischen oder organischen Säure, z.B. Salzsäure, Bromwasserstoffsäure, Phosphorsäure, Schwefelsäure, Essigsäure, Zitronensäure, Maleinsäure oder Benzoesäure um.

Die erfindungsgemässen Verbindungen der Formel I können in Form eines racemischen Gemisches anfallen. Die Trennung des Racemats in die optisch aktiven Formen geschieht nach an sich bekannten Methoden über die diastereomeren Salze. Als aktive Säuren können vorwiegend Weinsäure, Apfelsäure, Camphersäure und Camphersulfonsäure verwendet werden.

Zur Herstellung von Arzneimitteln werden die Verbindungen der Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemässen neuen Verbindungen der Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nichttoxische Salze), hochmolekulare Polymere (wie flüssiges Polyethylenoxyd) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole); für orale Applikation geeignete Zubereitungen können gewünschtenfalls Geschmacks- und Süssstoffe enthalten.

Die verabreichte Dosierung hängt vom Alter, der Gesundheit und dem Gewicht des Empfängers, dem Ausmass der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindungen 0,1–50 mg/kg Körpergewicht. Normalerweise sind 0,5–40 und vorzugsweise 1,0–20 mg/kg/Tag in einer oder mehreren Anwendungen pro Tag wirksam, um die gewünschten Resultate zu erhalten.

Neben den in den folgenden Beispielen aufgeführten Verbindungen sind im Sinne der vorliegenden Anmeldung weiterhin bevorzugt:

4-<2-Hydroxy-3-[2-(2,6-dimethoxy-phenoxy)-ethylamino]-propoxy>-7-methyl-3-propyl-2-benzimidazolinon

4-<2-Hydroxy-3-[2-(2-allyloxy-phenoxy)-ethylamino]-propoxy>-6-methylindol

4-<2-Hydroxy-3-[2-(2-methylmercapto-phenoxy)-ethylamino]-propoxy>-1-formylindolin

4-<2-Hydroxy-3-[2-(2-sulfamyl-phenoxy)-ethylamino]-propoxy>-indazol

4-<2-Hydroxy-3-[2-(3,4-ethylendioxy-phenyl)-ethylamino]-propoxy>-indazol

4-<2-Hydroxy-3-[2-(3-ethoxy-4-methoxy-phenyl)-ethylamino]-propoxy>-indazol

4-<2-Hydroxy-3-[2-(3,4,5-trimethoxy-phenyl)-ethylamino]-propoxy>-indazol

4-<2-Hydroxy-3-[2-(3-methoxy-4-hydroxy-phenyl)-ethylamino]-propoxy>-indazol

4-<2-Hydroxy-3-[2-(3-hydroxy-4-n-butoxy-phenyl)-ethylamino]-propoxy>-indazol

4-<2-Hydroxy-3-[2-(3,4-dihydroxy-phenyl)-ethylamino]-propoxy>-indazol

4-<2-Hydroxy-3-[3-(3,4-dimethoxy-phenyl)-propylamino]-propoxy>-indazol

4-<2-Hydroxy-3-[1-(3,4-dimethoxy-phenyl)-propyl-2-amino]-propoxy>-indazol

1-Methyl-4-<2-hydroxy-3-[2-(3,4-dimethoxy-phenyl)-ethylamino]-propoxy>-indazol

1-Methyl-4-<2-hydroxy-3-[2-(3,4-dimethoxy-phenyl)-ethylamino]-propoxy>-indolin

4-<2-Hydroxy-3-[2-(2-methoxy-phenylmercapto)-ethylamino]-propoxy>-1-formylindolin

4-<2-Benzoyloxy-3-[2-(3,4-dimethoxy-phenyl)-ethylamino]-propoxy>-indazol

4-<2-Hydroxy-3-[1-(3,4-dihydroxy-phenyl)-2-amino-1-propanol]-propoxy>-6-methylindol

Die nachfolgenden Beispiele zeigen einige der zahlreichen Verfahrensvarianten, die zur Synthese der erfindungsgemässen Verbindungen verwendet werden können. Sie sollen jedoch nicht eine Einschränkung des Erfindungsgegenstandes darstellen.

Beispiel 1

4-<2-Hydroxy-3-[-(2-methoxyphenoxy)-propylamino]-propoxy>-7-methyl-2-benzimidazolin-n-hydrochlorid

12,3 g 2,3-Diamino-1-<2-Hydroxy-3-[2-(2-methoxyphenoxy)-propylamino]-propoxy>-4-methyl-benzol-trihydrochlorid werden in 500 ml Wasser gelöst. Man leitet in diese Lösung 45 Minuten bei Raumtemperatur Phosgen ein, spült mit Stickstoff und saugt ab. Das Kristallisat wird aus 300 ml Ethanol/200 ml Methanol unter Zusatz von Aktivkohle umkristallisiert. Es werden 5,0 g (46% d. Th.) vom Fp. 228–230 °C erhalten.

Die hierzu benötigte Ausgangsverbindung wird folgendermassen erhalten:

30,5 g 2,3-Dinitro-1-(2,3-epoxy-propoxy)-4-methyl-benzol und 32,6 g N-Benzyl-2-(2-methoxyphenoxy)-propylamin werden in 500 ml Ethanol 3 Stunden unter Rückfluss gekocht. Das Reaktionsgemisch wird über 10 g 10%iger Palladiumkohle in 1 l Ethanol bei 50 °C und 30 bar hydriert. Man befreit vom Katalysator, säuert mit 2n Salzsäure an, klärt mit Aktivkohle und engt zur Trockne ein. Man erhält 59 g 2,3-Diamino-1-<2-hydroxy-3-[2-(2-methoxyphenoxy)-propylamino]-propoxy>-4-methyl-benzol-trihydrochlorid.

Beispiel 2

Analog Beispiel 1 werden aus Phosgen und dem entsprechend substituierten 1-Propoxy-2,3-diaminobenzol-Derivat erhalten:

| Bezeichnung | Ausb. % d. Th. | Fp. °C (Lösungsmittel) |
|---|---|---|
| a) 4-[2-Hydroxy-3-(3,4-dimethoxy-phenethyl-amino)propoxy]-7-methyl-2-benzimidazolinon-hydrochlorid aus 2,3-Diamino-1-[2-hydroxy-3-(3,4-dimethoxy-phenethyl-amino)propoxy]-4-methyl-benzol-trihydrochlorid | 42 | 204–206 (Isopropanol/ Methanol) |
| b) 4-<2-Hydroxy-3-[2-(2-hydroxyphenoxy)-ethylamino]-propoxy>-7-methyl-2-benzimidazolinon-hydrochlorid aus 2,3-Diamino-1-<2-hydroxy-3-[2-(2-hydroxyphenoxy)-ethylamino]propoxy>-4-methyl-benzol-trihydrochlorid | 67 | 262–263 (Methanol/ Wasser) |
| c) 6,7-Dimethyl-4-<2-hydroxy-3-[2-(2-methylphenoxy)-ethylamino]propoxy>2-benzimidazolinon-hydrochlorid aus 2,3-Diamino-4,5-dimethyl-1-<2-hydroxy-3-[2-(2-methylphenoxy)ethylamino]propoxy>-benzol-trihydrochlorid | 34 | 273–274 (Methanol/ Wasser) |
| d) 4-<2-Hydroxy-3-[2-(2-methoxyphenoxy)ethylamino]-propoxy>-7-methyl-2-benzimidazolinon-hydrochlorid aus 2,3-Diamino-1-<2-hydroxy-3-[2-(2-methoxyphenoxy)-ethylamino]propoxy>-4-methyl-benzol-trihydrochlorid | 62 | 202–203 (Ethanol/ Methanol) |

| Bezeichnung | Ausb. % d. Th. | Fp. °C (Lösungsmittel) |
|---|---|---|
| e) 4-[2-Hydroxy-3-(benzo[b]-1,4-dioxan-2-yl-methyl-amino)propoxy]-2-benzimidazolinon-hydrochlorid aus 2,3-Diamino-1-[2-hydroxy-3-(benzo[b]-1,4-dioxan-2-yl methylamino)propoxy]benzol-trihydrochlorid | | |
| f) 4-[2-Hydroxy-3-(2-phenoxy-ethylamino)propoxy]-7-methyl-2-benzimidazolinon-hydrochlorid aus 2,3-Diamino-1-[2-hydroxy-3-(2-phenoxy-ethylamino)-propoxy]-4-methyl-benzol-trihydrochlorid | 56 | 231–233 (Ethanol/ Methanol) |
| g) 4-[2-Hydroxy-3-(2-phenoxy-ethylamino)propoxy]-6-methyl-2-benzimidazolinon-hydrochlorid aus 2,3-Diamino-1-[2-hydroxy-3-(2-phenoxy-ethylamino)-propoxy]-5-methyl-benzol-trihydrochlorid | 52 | 250–252 (Ethanol/ Methanol) |
| h) 4-<2-Hydroxy-3-[2-(2-methoxy-phenoxy)ethylamino]-propoxy>-6-methyl-2-benzimidazolinon-hydrochlorid aus 2,3-Diamino-1-<2-hydroxy-3-[2-(2-methoxy-phenoxy)-ethylamino]propoxy>-5-methyl-benzol-trihydrochlorid | 87 | 213–215 (Ethanol) |
| i) 4-[2-Hydroxy-3-(3,4-dimethoxyphenethylamino)prop-oxy]-6-methyl-2-benzimidazolinon-hydrochlorid aus 2,3-Diamino-1-[2-hydroxy-3-(3,4-dimethoxyphenethyl-amino)propoxy]-5-methyl-benzol-trihydrochlorid | 15 | 260–262 (Ethanol) |
| j) 4-<2-Hydroxy-3-[2-(2-allyloxy-phenoxy)ethylamino]-propoxy>-7-methyl-2-benzimidazolinon-hydrochlorid aus 2,3-Diamino-1-<2-hydroxy-3-[2-(2-allyloxy-phenoxy)-ethylamino]propoxy>-4-methyl-benzol-trihydrochlorid | | |

Beispiel 3

4-<2-Hydroxy-3-[2-(2-methoxyphenoxy)-propyl-amino]-propoxy>-7-methyl-benzimidazolhydrochlorid

14,7 g 2,3-Diamino-1-<2-hydroxy-3-[2-(2-methoxyphenoxy)-propylamino]-propoxy>-4-methyl-benzol-trihydrochlorid werden in 80 ml Ameisensäure 3 Stunden rückfliessend gekocht. Man klärt mit Aktivkohle und engt zur Trockne ein. Die entstandene Formylverbindung wird in 50 ml 2n Salzsäure in der Siedehitze verseift. Nach Einengen zur Trockne wird der Rückstand aus 50 ml Ethanol unter Zusatz von Aktivkohle umkristallisiert. Man erhält 5,6 g (41% d. Th.) Titelverbindung vom Fp. 103–105 °C.

Beispiel 4

Analog zu Beispiel 3 erhält man aus Ameisensäure und dem entsprechend substituierten 1-Propoxy-2,3-diaminobenzol-Derivat:

| Bezeichnung | Ausb. % d. Th. | Fp. °C (Lösungsmittel) |
|---|---|---|
| a) 4-[2-Hydroxy-3-(3,4-dimethoxy-phenethylamino)prop-oxy]benzimidazol-hydrochlorid aus 2,3-Diamino-1-[2-hydroxy-3-(3,4-dimethoxyphenethyl-amino)propoxy]benzol-trihydrochlorid | 50 | 255–256 (Ethanol) |
| b) 4-[2-Hydroxy-3-(3,4-dimethoxy-phenethylamino)prop-oxy]-7-methyl-benzimidazol-hydrochlorid aus 2,3-Diamino-1-[2-hydroxy-3-(3,4-dimethoxy-phenethyl-amino)propoxy]-4-methyl-benzol-trihydrochlorid | 20 | 254–256 (Ethanol/ Wasser) |
| c) 4-<2-Hydroxy-3-[2-(2-hydroxyphenoxy)ethylamino]-propoxy>-7-methyl-benzimidazol-hydrochlorid aus 2,3-Diamino-1-<2-hydroxy-3-[2-(2-hydroxyphenoxy)-ethylamino]propoxy>-4-methyl-benzol-trihydrochlorid | 92 | 229–231 (Wasser) |
| d) 6,7-Dimethyl-4-<2-hydroxy-3-[2-(2-methyl-phenoxy)-ethylamino]propoxy> benzimidazol-hydrochlorid aus 2,3-Diamino-4,5-dimethyl-1-<2-hydroxy-3-[2-(2-methylphenoxy)ethylamino]propoxy> benzol-trihydrochlorid | 17 | 106–109 (Ethanol/ Essigester) |

| Bezeichnung | Ausb. % d. Th. | Fp. °C (Lösungsmittel) |
|---|---|---|
| e) 4-<2-Hydroxy-3-[2-(2-methoxy-phenoxy)ethylamino]-propoxy>-7-methyl-benzimidazol-hydrochlorid aus 2,3-Diamino-1-<2-hydroxy-3-[2-(2-methoxy-phenoxy)-ethylamino]propoxy>-4-methyl-benzol-trihydrochlorid | 62 | 219–221 Ethanol/ Methanol) |
| f) 4-<2-Hydroxy-3-[2-(2-methoxy-phenoxy)ethylamino]-propoxy>benzimidazol-hydrochlorid aus 2,3-Diamino-1-<2-hydroxy-3-[2-(2-methoxy-phenoxy)-ethylamino]propoxy>benzol-trihydrochlorid | 17 | 115–118 (Ethanol) |
| g) 4-[2-Hydroxy-3-(4-phenyl-2-butylamino)propoxy]-benzimidazol-hydrochlorid aus 2,3-Diamino-1-[2-hydroxy-3-(4-phenyl-2-butylamino)-propoxy]benzol-trihydrochlorid | 77 | amorph |
| h) 4-[2-Hydroxy-3-(2-phenoxy-ethylamino)propoxy]benzimidazol-hydrochlorid aus 2,3-Diamino-1-[2-hydroxy-3-(2-phenoxy-ethylamino)-propoxy]benzol-trihydrochlorid | 10 | amorph |
| i) 4-[2-Hydroxy-3-(benzo[b]-1,4-dioxan-2-yl-methyl-amino)-propoxy]benzimidazol-hydrochlorid aus 2,3-Diamino-1-[2-hydroxy-3-(benzo[b]-1,4-dioxan-2-yl-methylamino)propoxy]benzol-trihydrochlorid | 19 | 105–107 (Isopropanol/ Methanol) |
| j) 4-<2-Hydroxy-3-[2-(2-methoxy-phenoxy)propylamino]-propoxy>benzimidazol-hydrochlorid aus 2,3-Diamino-1-<2-hydroxy-3-[2-(2-methoxy-phenoxy)-propylamino]propoxy>benzol-trihydrochlorid | 59 | 115–116 (Ethanol) |

Beispiel 5

4-[2-Hydroxy-3-(3,4-dimethoxy-phenethylamino)-propoxy]-2,7-dimethyl-benzimidazol-hydrochlorid

14,6 g 2,3-Diamino-1-[2-hydroxy-3-(3,4-dimethoxy-phenethylamino)-propoxy]-4-methyl-benzol-trihydrochlorid werden in 50 ml Eisessig 3 Stunden unter Rückfluss erhitzt. Es wird mit Aktivkohle geklärt und vollständig eingeengt. Der Rückstand wird in 40 ml 2n Salzsäure 2 Stunden unter Rückfluss gekocht und anschliessend zur Trockne eingeengt. Man reinigt über eine Kieselgelsäule mit Chloroform/Methanol 8 : 2 als Elutionsmittel und kristallisiert schliesslich aus 100 ml Ethanol 2,2 g (19% d. Th.) Titelverbindung vom Fp. 167–169 °C.

Das als Ausgangsmaterial eingesetzte Diamin wird folgendermassen erhalten:

26,0 g N-Benzyl-3,4-dimethoxy-phenethylamin und 24,3 g 2,3-Dinitro-1-(2,3-epoxy-propoxy)-4-methyl-benzol werden in 300 ml Ethanol 4 Stunden unter Rückfluss gekocht. Die Reaktionsmischung wird mit 300 ml Ethanol verdünnt und bei 50 °C und 30 bar Druck über 7,0 g 10%iger Palladiumkohle hydriert. Man befreit vom Katalysator, säuert mit 2n Salzsäure an und erhält nach Einengen das amorphe Diaminsalz.

Beispiel 6

Analog zu Beispiel 5 wird aus Essigsäure und entsprechend substituierten 1-Propoxy-2,3-diaminobenzol-Derivaten erhalten:

| Bezeichnung | Ausb. % d. Th. | Fp. °C (Lösungsmittel) |
|---|---|---|
| a) 4-<2-Hydroxy-3-[2-(2-propoxy-phenoxy)ethylamino]-propoxy>-2,7-dimethyl-benzimidazol-hydrochlorid aus 2,3-Diamino-4-methyl-1-<2-hydroxy-3-[2-(2-propoxy-phenoxy)-ethylamino]propoxy>benzol-trihydrochlorid | 44 | 143–145 (Alkohol/ Essigester) |
| b) 4-<2-Hydroxy-3-[2-(2-methoxy-4-methyl-phenoxy)-ethylamino]propoxy>-2,7-dimethyl-benzimidazol-hydrochlorid aus 2,3-Diamino-4-methyl-1-<2-hydroxy-3-[2-(2-methoxy-4-methyl-phenoxy)ethylamino]propoxy>benzol-trihydrochlorid | 62 | 165–167 (Ethanol) |

Beispiel 7

4-[2-Hydroxy-3-(3,4-dimethoxy-phenethylamino)-propoxy]-benzo-triazol-hydrochlorid

14,1 g 2,3-Diamino-1-[2-hydroxy-3-(3,4-dimethoxy-phenethylamino)-propoxy]-benzol-trihydrochlorid werden in 10 ml Wasser und 3,5 ml Eisessig gelöst, auf 5 °C abgekühlt und mit 2,07 g Natriumnitrit in 3,6 ml Wasser versetzt. Man rührt 2,5 Stunden bei Raumtemperatur nach. Durch Zugabe von 480 ml Isopropanol wird das entstandene Natriumchlorid ausgefällt. Nach Filtration wird eingeengt, über eine Kieselgelsäule (Laufmittel Methanol/Essigester 2 : 1) gereinigt, mit etherischer Salzsäure versetzt und aus Ethanol kristallisiert. Man erhält 1,26 g (10% d. Th.) Titelverbindung vom Fp. 123–124 °C.

Beispiel 8

Analog zu Beispiel 7 werden aus Natriumnitrit und dem entsprechend substituierten 1-Propoxy-2;3-diaminobenzol-Derivat erhalten:

| Bezeichnung | Ausb. % d. Th. | Fp. °C (Lösungsmittel) |
|---|---|---|
| a) 4-[2-Hydroxy-3-(3,4-dimethoxy-phenethylamino)propoxy]-7-methyl-benzotriazol-hydrochlorid aus 2,3-Diamino-1-[2-hydroxy-3-(3,4-dimethoxy-phenethylamino)propoxy]-4-methyl-benzol-trihydrochlorid | 16 | 184–186 (Isopropanol) |
| b) 4-<2-Hydroxy-3-[2-(2-methoxyphenoxy)ethylamino]-propoxy>benzotriazol-hydrochlorid aus 2,3-Diamino-1-<2-hydroxy-3-[2-(2-methoxyphenoxy)-ethylamino]propoxy>benzol-trihydrochlorid | 25 | 113–115 (Methanol/ Ether) |
| c) 4-<2-Hydroxy-3-[2-(2-methoxyphenoxy)ethylamino]-propoxy>-7-methyl-benzotriazol-hydrochlorid aus 2,3-Diamino-1-<2-hydroxy-3-[2-(2-methoxyphenoxy)-ethylamino]propoxy>-4-methyl-benzol-trihydrochlorid | 48 | 109–111 (Isopropanol/ Methanol) |
| d) 4-<2-Hydroxy-3-[2-(2-hydroxyphenoxy)ethylamino]-propoxy>benzotriazol-hydrochlorid aus 2,3-Diamino-1-<2-hydroxy-3-[2-(2-hydroxyphenoxy)-ethylamino]propoxy>benzol-trihydrochlorid | 14 | 200–203 (Ethanol/ Methanol |
| e) 4-[2-Hydroxy-3-(2-methoxy-phenethyl-amino)propoxy]-benzotriazol-hydrochlorid aus 2,3-Diamino-1-[2-hydroxy-3-(2-methoxy-phenethyl-amino)propoxy]benzol-trihydrochlorid | 21 | 82–85 (Alkohol/ Essigester) |
| f) 4-<2-Hydroxy-3-[2-(2-methoxy-phenoxy)propylamino]-propoxy>benzotriazol-hydrochlorid aus 2,3-Diamino-1-<2-hydroxy-3-[2-(2-methoxy-phenoxy)-propylamino]propoxy>benzol-trihydrochlorid | 62 | amorph |
| g) 4-<2-Hydroxy-3-[2-(2-allyloxyphenoxy)-propylamino]-propoxy>benzotriazol-hydrochlorid aus 2,3-Diamino-1-<2-hydroxy-3-[2-(2-allyloxy-phenoxy)-propylamino]propoxy>benzol-trihydrochlorid | 15 | 143–145 (Ethanol/ Essigester) |

Beispiel 9

4-[2-Hydroxy-3-(3,4-dimethoxy-phenethylamino)-propoxy]-2-benzimidazolinthion-hydrochlorid

17,4 g 2,3-Diamino-1-[2-hydroxy-3-(3,4-dimethoxy-phenethylamino)-propoxy]-benzol-trihydrochlorid werden in 450 ml Chloroform und 50 ml Ethanol gelöst und bei Raumtemperatur mit 3,2 ml Thiophosgen in 30 ml Chloroform tropfenweise versetzt.

Nach 2 Stunden wird mit Aktivkohle behandelt und zur Trockne eingeengt. Man reinigt über eine Kieselgelsäule (Chloroform/Methanol 8 : 2) und erhält 10 g (62% d. Th.) einheitliches Produkt. Nach Aufnehmen in Isopropanol und Versetzen mit etherischer Salzsäure wird mit Essigester die Titelverbindung vom Fp. 108–110 °C ausgefällt.

Beispiel 10

Analog Beispiel 9 werden aus Thiophosgen und dem entsprechend substituierten 1-Propoxy-2,3-diaminobenzol-Derivat erhalten:

| Bezeichnung | Ausb. % d. Th. | Fp. °C (Lösungsmittel) |
|---|---|---|
| a) 4-[2-Hydroxy-3-(3,4-dimethoxy-phenethylamino)propoxy]-7-methyl-2-benzimidazolinthion-hydrochlorid aus 2,3-Diamino-1-[2-hydroxy-3-(3,4-dimethoxy-phenethylamino)propoxy]-4-methyl-benzol-trihydrochlorid | | |

| Bezeichnung | Ausb. % d. Th. | Fp. °C (Lösungsmittel) |
|---|---|---|
| b) 4-<2-Hydroxy-3-[2-(2-methoxyphenoxy)propylamino]-propoxy>-7-methyl-2-benzimidazolinthionbenzoat aus 2,3-Diamino-1-<2-hydroxy-3-[2-(2-methoxyphenoxy)-propylamino]propoxy>-4-methyl-benzol-trihydrochlorid | 14 | 169–170 (Ethanol/ Ether) |
| c) 4-<2-Hydroxy-3-[2-(2-methylphenoxy)ethylamino]-propoxy>-2-benzimidazolinthion-hydrochlorid aus 2,3-Diamino-1-<2-hydroxy-3-[2-(2-methylphenoxy)-ethylamino]propoxy>benzol-trihydrochlorid | | |

Beispiel 11

4-<2-Hydroxy-3-[2-hydroxy-phenoxy)-ethylamino]-propoxy>-6,7-cyclopenteno-2-benzimidazolinon-hydrochlorid

In die Lösung von 6,5 g (0,0175 mol) 4,5-Diamino-6-<2-hydroxy-3-[2-(2-hydroxy-phenoxy)-ethylamino]-propoxy>-indan-hydrochlorid in 250 ml Wasser und 100 ml Tetrahydrofuran leitet man 1 Stunde Phosgen ein, spült gründlich mit Stickstoff nach, filtriert, nimmt den Niederschlag in verdünnter Natronlauge auf, wäscht mit Methylenchlorid, stellt die wässrige Phase auf pH 7 ein, extrahiert mit Methylenchlorid, engt ein, nimmt in Aceton auf und fällt mit etherischer Salzsäure das Hydrochlorid aus. Man erhält 2,8 g Titelverbindung (36% d. Th.) als amorphes Salz vom Schmp. 145–155 °C.

Die als Ausgangsprodukt verwendete Diaminoverbindung kann auf folgendem Weg hergestellt werden:

Die Kondensation von 5-Amino-6-(2,3-epoxy-propoxy)-4-nitro-indan und N-Benzyl-2-(2-benzyloxy-phenoxy)-ethylamin liefert 5-Amino-6-<3-[N-benzyl-2-(2-benzyloxy-phenoxy)-ethylamino]-2-hydroxy-propoxy>-4-nitro-indan als Öl.

Durch Hydrierung und hydrogenolytische Debenzylierung der vorstehenden Verbindung als Hydrochlorid in methanolischer Lösung über 10%iger Palladiumkohle gelangt man zu 4,5-Diamino-6-<2-hydroxy-3-[2-(2-hydroxy-phenoxy)-ethylamino]-propoxy>-indan-hydrochlorid vom Schmp. 193–195 °C.

Beispiel 12

4-<3-[2-(3,4-Dimethoxy-phenyl)-ethylamino]-2-hydroxy-propoxy>-6,7-cyclopenteno-2-benzimidazolinon-hydrochlorid

Analog dem in Beispiel 1 beschriebenen Verfahren erhält man aus Phosgen und 4,5-Diamino-6-<3-[2-(3,4-dimethoxy-phenyl)-ethylamino]-2-hydroxy-propoxy>-indan-hydrochlorid die Titelverbindung als Hydrochlorid vom Schmp. 193–195 °C.

Die als Ausgangsprodukt verwendete Diaminoverbindung kann auf folgendem Weg hergestellt werden:

Durch Reaktion von 5-Amino-6-(2,3-epoxy-propoxy)-4-nitro-indan und 3,4-Dimethoxy-phenethylamin erhält man 5-Amino-6-<3-[2-(3,4-dimethoxy-phenyl)-ethylamino]-2-hydroxy-propoxy>-4-nitro-indan vom Schmp. 230–236 °C (als Hydrochlorid).

Die Hydrierung der vorstehenden Verbindung in methanolischer Lösung über Platindioxid liefert 4,5-Diamino-6-<3-[2-(3,4-dimethoxy-phenyl)-ethylamino-2-hydroxy-propoxy>-indan, das als amorphes Hydrochlorid isoliert wird.

Beispiel 13

4-[2-Hydroxy-3-(2-phenoxy-propylamino)-propoxy]-6,7-cyclopenteno-2-benzimidazolinon-hydrochlorid

Analog dem in Beispiel 1 beschriebenen Verfahren erhält man aus Phosgen und 4,5-Diamino-6-[2-hydroxy-3-(2-phenoxy-propylamino)-propoxy]-indan-hydrochlorid die Titelverbindung als Hydrochlorid vom Schmp. 261–263 °C.

Die als Ausgangsprodukt verwendete Diaminoverbindung lässt sich wie folgt herstellen:

Die Kondensation von 5-Amino-6-(2,3-epoxy-propoxy)-4-nitro-indan und N-Benzyl-2-phenoxy-propylamin liefert 5-Amino-6-[3-(N-benzyl-2-phenoxy-propylamino)-2-hydroxy-propoxy]-4-nitro-indan vom Schmp. 70–80 °C (als amorphes Hydrochlorid).

Durch Hydrierung und hydrogenolytische Debenzylierung der vorstehenden Verbindung in methanolischer Lösung über 10%iger Palladiumkohle gelangt man zu 4,5-Diamino-6-[2-hydroxy-3-(2-phenoxy-propylamino)-propoxy]-indan-hydrochlorid vom Schmp. 153–155 °C.

Beispiel 14

4-<2-Hydroxy-3-[2-(2-hydroxy-phenoxy)-ethyl-amino]-propoxy>-6,7-cyclopenteno-benzimidazol

Eine Mischung aus 4,1 g (0,01 mol) 4,5-Diamino-6-<2-hydrooxy-3-[2-(2-hydroxy-phenoxy)-ethyl-amino]-propoxy>-indan-hydrochlorid und 35 ml Formamid wird 40 Minuten zum Rückfluss erhitzt, eingeengt, mit Wasser versetzt, mit Methylenchlorid extrahiert, der Extrakt eingeengt und mit Essigester verrieben. Man erhält 1,3 g Titelverbindung (34% d. Th.) vom Schmp. 182–183 °C.

Beispiel 15

4-<2-Hydroxy-3-[(1-methyl-3-phenyl)-propyl-amino]-propoxy>-6-methylindol-benzoat

4,9 g 4-(2,3-Epoxy-propoxy)-6-methylindol (DE-OS 2 508 251) und 3,7 g (1-Methyl-3-phenyl)-propylamin werden in 50 ml n-Butanol 18 Stunden gerührt, im Vakuum eingeengt und mit 1n Milchsäure und Ether geschüttelt. Nach Ausfällen der Base mit verdünnter Natronlauge wird mit Ether/Essigester 1 : 1 extrahiert und über Kieselgel mit Methylenchlorid/Methanol 9 : 1 chromatographisch gereinigt. Die erhaltene Base wird in Essigester gelöst, mit der äquivalenten Menge Benzoesäure versetzt und abgesaugt. Man erhält 2,0 g 4-<2-Hydroxy-3-[(1-methyl-3-phenyl)-propylamino]-propoxy>-6-methylindol-benzoat vom Schmp. 119–122 °C (18% d. Th.).

Beispiel 16

In analoger Weise, wie in Beispiel 15 beschrieben, erhält man:

| Bezeichnung | Ausb. % d. Th. | Smp. °C (Lösungsmittel) |
|---|---|---|
| a) 4-<2-Hydroxy-3-[2-(3,4-dimethoxy-phenyl)ethylamino]propoxy>-6-methylindol-benzoat aus 4-(2,3-Epoxy-propoxy)-6-methylindol und 2-(3,4-Dimethoxy-phenyl)ethylamin | 27 | 147–148 (Essigester) |
| b) 4-<2-Hydroxy-3-[2-(2-pyridinyl)ethylamino]propoxy>-6-methylindol-di-p-nitrobenzoat aus 4-(2,3-Epoxy-propoxy)-6-methylindol und 2-(2-Pyridinyl)ethylamin | 16 | 146 (Methanol) |
| c) 4-[2-Hydroxy-3-(2-phenoxy-ethylamino)propoxy]-6-methyl-indol-benzoat aus 4-(2,3-Epoxy-propoxy)-6-methylindol und 2-Phenoxy-ethylamin | 16 | 123–125 (Ether) |
| d) 4-<2-Hydroxy-3-[(1-methyl-2-phenyl)-ethylamino]-propoxy>-6-methylindol-benzoat aus 4-(2,3-Epoxy-propoxy)-6-methylindol und (1-Methyl-2-phenyl)-ethylamin | 20 | 125–128 (Essigester) |
| e) 4-<2-Hydroxy-3-[2-(2-methoxy-phenoxy)ethylamino]-propoxy>-6-methylindol aus 4-(2,3-Epoxy-propoxy)-6-methylindol und 2-(2-Methoxyphenoxy)ethylamin | 19 | 123–125 (Essigester) |
| f) 4-<2-Hydroxy-3-[2-(4-carbamido-phenoxy)ethylamino]-propoxy>-6-methylindol aus 4-(2,3-Epoxy-propoxy)-6-methylindol und 2-(4-Carbamido-phenoxy)ethylamin | 19 | 128–130 (Methanol) |
| g) 4-[2-Hydroxy-3-(benzo[b]-1,4-dioxan-2-yl-methylamino)-propoxy]-6-methylindol-p-chlorbenzoat aus 4-(2,3-Epoxy-propoxy)-6-methylindol und 2-(Aminomethyl)-1,4-benzo[b]dioxan | 14 | 168–170 (Essigester) |
| h) 4-[2-Hydroxy-3-(2-phenoxy-N-benzyl-propylamino)-propoxy]-6-methylindol aus 4-(2,3-Epoxy-propoxy)-6-methylindol und 2-Phenoxy-N-benzyl-propylamin | 95 | Öl |
| i) 4-<2-Hydroxy-3-[2-(2-methoxy-phenoxy)-N-benzyl-propyl-amino]propoxy>-6-methylindol aus 4-(2,3-Epoxy-propoxy)-6-methylindol und 2-(2-Methoxy-phenoxy)-N-benzyl-propylamin | 95 | Oel |
| j) 4-<2-Hydroxy-3-[2-(5-carbamido-2-pyridoxy)-N-benzyl-ethylamino]propoxy>-6-methylindol aus 4-(2,3-Epoxy-propoxy)-6-methylindol und 2-(5-Carbamido-2-pyridoxy)-N-benzyl-ethylamin | 76 | Öl |
| k) 4-<2-Hydroxy-3-[2-(2-benzyloxy-phenoxy)ethylamino]propoxy>-6-methylindol aus 4-(2,3-Epoxy-propoxy)-6-methylindol und 2-(2-Benzyloxy-phenoxy)ethylamin | 16 | Öl |
| l) 4-<2-Hydroxy-3-[2-(3,4-dimethoxy-phenyl)ethylamino]propoxy>-6-methoxycarbonylindol aus 4-(2,3-Epoxy-propoxy)-6-methoxycarbonylindol und 2-(3,4-Dimethoxy-phenyl)ethylamin | 59 | 145–147 (Isopropanol/Ether) |
| m) 4-<2-Hydroxy-3-[2-(3,4-dimethoxy-phenyl)ethylamino]propoxy>-6-hydroxymethylindol aus 4-(2,3-Epoxy-propoxy)-6-hydroxymethylindol und 2-(3,4-Dimethoxy-phenyl)ethylamin | 35 | 60 (amorph) |

| Bezeichnung | Ausb. % d. Th. | Smp. °C (Lösungsmittel) |
|---|---|---|
| n) 4-<2-Hydroxy-3-[2-(2-allyloxy-phenoxy)ethylamino]-propoxy>-6-methylindol aus 4-(2,3-Epoxy-propoxy)-6-methylindol und 2-(Allyloxy-phenoxy)ethylamin | 36 | 125 (Methanol) |

Beispiel 17

4-[2-Hydroxy-3-(2-phenoxy-propylamino)-propoxy]-6-methylindol-p-chlorbenzoat

Man hydriert 8 g 4-[2-Hydroxy-3-(2-phenoxy-N-benzyl-propylamino)-propoxy]-6-methylindol (Herstellung siehe Beispiel 16h) in 200 ml Methanol und 5 ml Triethylamin bei Raumtemperatur und 1 bar Wasserstoffdruck über 2 g 10%iger Palladiumkohle, filtriert, engt ein, löst den verbleibenden Rückstand in 50 ml Essigester und versetzt mit der berechneten Menge p-Chlorbenzoesäure. Nach Absaugen und Umkristallisieren erhält man 4,3 g 4-[2-Hydroxy-3-(2-phenoxy-propylamino)-propoxy]-6-methylindol-p-chlorbenzoat vom Schmp. 134–136 °C (42% d. Th.).

Beispiel 18

In analoger Weise, wie in Beispiel 17 beschrieben, erhält man:

| Bezeichnung | Ausb. % d. Th. | Smp. °C (Lösungsmittel) |
|---|---|---|
| a) 4-<2-Hydroxy-3-[2-(2-methoxy-phenoxy)propylamino] propoxy>-6-methylindol aus 4-<2-Hydroxy-3-[2-(2-methoxy-phenoxy)-N-benzyl-propylamino]propoxy>-6-methylindol (s. Bsp.16i) | 58 | 104–106 (Essigester) |
| b) 4-<2-Hydroxy-3-[2-(5-carbamido-2-pyridoxy)ethylamino]propoxy>-6-methylindol-di-p-chlorbenzoat aus 4-<2-Hydroxy-3-[2-(5-carbamido-2-pyridoxy)-N-benzyl-ethylamino]propoxy>-6-methylindol (s. Bsp. 16j) | 10 | 141–143 (Isopropanol) |
| c) 4-<2-Hydroxy-3-[2-(2-hydroxy-phenoxy)ethylamino]-propoxy>-6-methylindol-benzoat aus 4-<2-Hydroxy-3-[2-(2-benzyloxy-phenoxy)ethylamino]propoxy>-6-methylindol (s. Bsp. 16k) | 72 | 85–90 (Methanol) |

Beispiel 19

4-<2-Hydroxy-3-[2-(3,4-dimethoxy-phenyl)-ethylamino]-propoxy>-6-hydroxymethylindol

Zu einer Suspension von 2,3 g Lithiumaluminiumhydrid in 50 ml absolutem Tetrahydrofuran tropft man eine Lösung von 5,0 g 4-<2-Hydroxy-3-[2-(3,4-dimethoxy-phenyl)-ethylamino]-propoxy->-6-methoxycarbonylindol (Herstellung siehe Beispiel 16i) in 150 ml absolutem Tetrahydrofuran, rührt 12 Stunden bei Raumtemperatur nach, zersetzt unter Kühlung mit Natriumchlorid-Lösung und 10n Natronlauge, filtriert, wäscht mit Tetrahydrofuran nach und engt ein. Der Rückstand wird über eine Kieselgelsäure mit Methylenchlorid/Methanol 9 : 1 gereinigt. Die Base wird mit Ether ausgefällt und abgesaugt. Es werden 2,4 g 4-<2-Hydroxy-3-[2-(3,4-dimethoxy-phenyl)-ethylamino]-propoxy>-6-hydroxymethylindol vom Sinterpunkt 60 °C erhalten (51% d. Th.).

Beispiel 20

2-Ethoxycarbonyl-4-[2-hydroxy-3-(3-phenyl-propylamino)-propoxy]-6-methylindol

3,6 g 2-Ethoxycarbonyl-4-(2,3-epoxy-propoxy)-6-methylindol (DE-OS 2 626 890) und 1,76 g 3-Phenyl-propylamin werden in 5 ml Hexamethylphosphorsäuretriamid bis zur völligen Umsetzung bei Raumtemperatur gerührt, in Wasser und Ether aufgenommen, die Etherphase mit 1n Weinsäure extrahiert und die Base mit Kaliumcarbonatlösung freigesetzt. Nach Ausschütteln mit Ether, Trocknen und Eindampfen verbleiben 2,0 g 2-Ethoxycarbonyl-4-[2-hydroxy-3-(3-phenyl-propylamino)-propoxy]-6-methylindol vom Schmp. 127 °C (33% d. Th.).

Herstellung des Acetats:

1,4 g 2-Ethoxycarbonyl-4-[2-hydroxy-3-(3-phenyl-propylamino)-propoxy]-6-methylindol werden unter leichtem Erwärmen in 10 ml Essigester gelöst, mit 0,2 ml Essigsäure versetzt und nach Erkalten abgesaugt. Man erhält 1,3 g 2-Ethoxycarbonyl-4-[2-hydroxy-3-(3-phenyl-propylamino)-propoxy]-6-methylindol als Acetat vom Schmp. 155°C.

Beispiel 21

In analoger Weise, wie in Beispiel 20 beschrieben, erhält man:

| Bezeichnung | Ausb. % d. Th. | Smp. °C (Lösungsmittel) |
|---|---|---|
| a) 2-Ethoxycarbonyl-4-<2-hydroxy-3-[(1-methyl-3-phenyl)-propyl-amino]propoxy>-6-methylindol aus 2-Ethoxycarbonyl-4-(2,3-epoxy-propoxy)-6-methylindol und (1-Methyl-3-phenyl)-propylamin | 28 | 133–134 (Essigester) |
| b) 2-Ethoxycarbonyl-4-<2-hydroxy-3-[2-(2-pyridinyl)-ethylamino]propoxy>-6-methylindol-benzoat aus 2-Ethoxycarbonyl-4-(2,3-epoxy-propoxy)-6-methylindol und 2-(2-Pyridinil)ethylamin | 20 | 129–131 (Essigester) |
| c) 2-Ethoxycarbonyl-4-<2-hydroxy-3-[2-(4-pyridinyl)-ethylamino]propoxy>-6-methylindol aus 2-Ethoxycarbonyl-4-(2,3-epoxy-propoxy)-6-methylindol und 2-(4-Pyridinyl)ethylamin | 32 | 133–134 (Ether) |
| d) 2-Ethoxycarbonyl-4-[2-hydroxy-3-(2-phenoxy-ethylamino)propoxy]-6-methylindol-benzoat aus 2-Ethoxycarbonyl-4-(2,3-epoxy-propoxy)-6-methylindol und 2-Phenoxy-ethylamin | 24 | 147 (Essigester) |
| e) 2-Ethoxycarbonyl-4-<2-Hydroxy-3-[(1-methyl-2-phenoxy)ethylamino]propoxy>-6-methylindol-benzoat aus 2-Ethoxycarbonyl-4-(2,3-epoxy-propoxy)-6-methylindol und 1-Methyl-2-phenoxy-ethylamin | 23 | 135–137 (Ether) |
| f) 2-Ethoxycarbonyl-4-<2-hydroxy-3-[2-(2-methoxy-phenoxy)ethylamino]propoxy>-6-methylindol aus 2-Ethoxycarbonyl-4-(2,3-epoxy-propoxy)-6-methylindol und 2-(2-Methoxy-phenoxy)ethylamin | 21 | 145–147 (Essigester) |
| g) 2-Ethoxycarbonyl-4-<2-hydroxy-3-[2-(4-carbamido-phenoxy)ethylamino]propoxy>-6-methylindol-acetat aus 2-Ethoxycarbonyl-4-(2,3-epoxy-propoxy)-6-methylindol und 2-(4-Carbamido-phenoxy)ethylamin | 18 | 160–165 (Essigester) |
| h) 2-Ethoxycarbonyl-4-[2-hydroxy-3-(benzo[b]-1,4-dioxan-2-yl-methylamino)propoxy]-6-methylindol aus 2-Ethoxycarbonyl-4-(2,3-epoxy-propoxy)-6-methylindol und 2-(Aminomethyl)-1,4-benzo[b]dioxan | 25 | 135–137 (Ether) |

Beispiel 22

4-[2-Hydroxy-3-(3-phenyl-propylamino)-propoxy]-indazol

5 g 4-(2,3-Epoxy-propoxy)-indazol und 25 g 3-Phenyl-propylamin werden 20 Stunden bei 50 °C gerührt. Man destilliert überschüssiges Amin im Vakuum ab, reibt den Rückstand mit Ether an, saugt ab und kristallisiert aus Essigester um. Es werden 4,3 g 4-[2-Hydroxy-3-(3-phenylpropylamino)-propoxy]-indazol vom Schmp. 122–124 °C erhalten (50% d. Th.).

Beispiel 23

4-<2-Hydroxy-3-[(1-methyl-2-phenoxy)-äthylamino]-propoxy>-indazol

5 g 4-(2,3-Epoxy-propoxy)-indazol und 4,17 g (1-Methyl-2-phenoxy-Ethyl)-amin werden in 10 ml 1,2-Dimethoxyethan 20 Stunden auf 50 °C erwärmt. Nach Anreiben mit Aceton, Absaugen und Umkristallisieren aus Isopropanol erhält man 3,7 g 4-<2-Hydroxy-3-[(1-methyl-2-phenoxy)-ethylamino]-propoxy>-indazol vom Schmp. 175–177 °C (41% d. Th.).

Beispiel 24

2-Benzyl-4-<2-hydroxy-3-[2-(3,4-dimethoxy-phenyl)-ethylamino]-propoxy>-indazol

17 g 2-benzyl-4-(2,3-epoxy-propoxy)-indazol und 20 g 2-(3,4-dimethoxy-phenyl)-ethylamin werden 20 Stunden bei 70 °C gerührt. Man löst überschüssiges Amin durch Ausrühren mit Ether und saugt ab. Es werden 19,1 g Titelverbindung vom Schmp. 101–102 °C erhalten (74% d. Th.).

Beispiel 25

In analoger Weise, wie in Beispiel 24 beschrieben, erhält man:

| Bezeichnung | Ausb. % d. Th. | Smp. °C (Lösungsmittel) |
|---|---|---|
| 2-Benzyl-4-<2-hydroxy-3-[2-(2-benzyloxy-phenoxy)-ethylamino]propoxy>indazol aus 2-Benzyl-4-(2,3-epoxy-propoxy)indazol und 2-(2-Benzyloxy-phenoxy)ethylamin | 91 | 81–83 (Ethanol) |

Beispiel 26

2-Benzyl-4-[2-hydroxy-3-(2-phenoxy-ethylamino)-propoxy]-indazol

5 g 2-Benzyl-4-(2,3-epoxy-propoxy)-indazol und 2,6 g 2-Phenoxy-ethylamin werden in 10 ml 1,2-Dimethoxyethan 48 Stunden auf 50 °C erwärmt. Man engt ein, löst in Methylenchlorid und reinigt chromatographisch über eine Kieselgelsäule mit den Laufmitteln Methylenchlorid/Essigester 8 : 2 und Essigester. Nach Eindampfen werden 4,2 g Titelverbindung als viskoses Öl erhalten (56% d. Th.).

Beispiel 27

In analoger Weise, wie in Beispiel 26 beschrieben, erhält man:

| Bezeichnung | Ausb. % d. Th. | Smp. °C (Lösungsmittel) |
|---|---|---|
| a) 2-Benzyl-4-<2-hydroxy-3-[2-(2-methoxy-phenoxy)-ethylamino]propoxy>indazol aus 2-Benzyl-4-(2,3-epoxy-propoxy)indazol und 2-(2-Methoxy-phenoxy)ethylamin | 52 | Öl |
| b) 2-Benzyl-4-<2-hydroxy-3-[2-(2-methoxy-phenoxy)-propylamino]propoxy>indazol aus 2-Benzyl-4-(2,3-epoxy-propoxy)indazol und 2-(Methoxy-phenoxy)propylamin | 83 | Öl |
| c) 2-Benzyl-4-<2-hydroxy-3-[2-(4-pyridil)ethylamino]-propoxy>indazol aus 2-Benzyl-4-(2,3-epoxy-propoxy)indazol und 2-(4-Pyridil)ethylamin | 51 | Öl |
| d) 2-Benzyl-4-[2-hydroxy-3-(benzo[b]-1,4-dioxan-2-yl-methylamino)propoxy]indazol aus 2-Benzyl-4-(2,3-epoxy-propoxy)indazol und 2-(Aminomethyl)-1,4-benzo[b]dioxan | 47 | Öl |
| e) 2-Benzyl-4-<2-hydroxy-3-[2-(3,4-ethylendioxyphenyl)-ethylamino]propoxy>indazol aus 2-Benzyl-4-(2,3-epoxy-propoxy)indazol und 2-(3,4-Ethylendioxyphenyl)ethylamin | 76 | 165–168 (Isopropanol) |
| f) 2-Benzyl-4-<2-hydroxy-3-[2-(3-ethoxy-4-methoxy-phenyl)ethylamino]propoxy>indazol aus 2-Benzyl-4-(2,3-epoxy-propoxy)indazol und 2-(3-Ethoxy-4-methoxy-phenyl)ethylamin | 85 | Öl |
| g) 2-Benzyl-4-<2-hydroxy-3-[2-(3,4,5-trimethoxyphenyl)-ethylamino]propoxy>indazol aus 2-Benzyl-4-(2,3-epoxy-propoxy)indazol und 2-(3,4,5-Trimethoxyphenyl)ethylamin | 88 | Öl |
| h) 2-Benzyl-4-<2-hydroxy-3-[2-(4-benzyloxy-3-methoxy-phenyl)ethylamino]propoxy>-indazol aus 2-Benzyl-4-(2,3-epoxy-propoxy)indazol und 2-(4-Benzyloxy-3-methoxy-phenyl)ethylamin | 78 | 96–98 (Methanol) |
| i) 2-Benzyl-4-<2-hydroxy-3-[2-(3-benzyloxy-4-butoxy-phenyl)ethylamino]propoxy>indazol aus 2-Benzyl-4-(2,3-epoxy-propoxy)indazol und 2-(3-Benzyloxy-4-butoxy-phenyl)ethylamin | 80 | Öl |

| Bezeichnung | Ausb. % d. Th. | Smp. °C (Lösungsmittel) |
|---|---|---|
| j) 2-Benzyl-4-<2-hydroxy-3-[2-(3,4-dibenzyloxyphenyl)-ethylamino]propoxy>indazol aus 2-Benzyl-4-(2,3-epoxy-propoxy)indazol und 2-(3,4-Dibenzyloxy-phenyl)ethylamin | 84 | Öl |
| k) 2-Benzyl-4-<2-hydroxy-3-[2-(2-pyridoxy)ethylamino]-propoxy>indazol aus 2-Benzyl-4-(2,3-epoxy-propoxy)indazol und 2-(2-Pyridoxy)ethylamin | 64 | Öl |

Beispiel 28

2-Benzyl-4-<2-hydroxy-3-[2-(2-hydroxy-phenoxy)-N-benzyl-propylamino]-propoxy>-indazol

4,2 g 2-Benzyl-4-(2,3-epoxy-propoxy)-indazol und 3,85 g 2-(2-Hydroxy-phenoxy)-N-Benzyl-propylamin werden in 30 ml Dioxan 4d unter Stickstoff am Rückfluss gekocht. Man engt ein, löst in Methylenchlorid und reinigt chromatographisch über eine Kieselgelsäule. Nach Eindampfen verbleiben 5,8 g Titelverbindung als viskoses Öl (72% d. Th.).

Beispiel 29

In analoger Weise, wie in Beispiel 23 beschrieben, erhält man:

| Bezeichnung | Ausb. % d. Th. | Smp. °C (Lösungsmittel) |
|---|---|---|
| 2-Benzyl-4-<2-hydroxy-3-[2-(4-acetamido-phenoxy)-N-benzyl-ethylamino]propoxy>indazol aus 2-Benzyl-4-(2,3-epoxy-propoxy)indazol und 2-(4-Acetamido-phenoxy)-N-benzyl-ethylamin | 67 | Öl |

Das als Ausgangsprodukt verwendete 2-Benzyl-4-(2,3-epoxy-propoxy)-indazol kann wie folgt erhalten werden:

Man trägt unter Kühlen und Stickstoffatmosphäre 9,2 g Natriumhydrid-Paraffin (55–60%ige Suspension) in eine Lösung von 47,1 g 2-Benzyl-4-hydroxyindazol in 250 ml Dimethylformamid ein, tropft nach beendeter $H_2$-Entwicklung 19 ml Epibromhydrin zu und rührt 16 Stunden bei Raumtemperatur. Danach rührt man in 1,5 l Wasser ein, extrahiert mit Methylenchlorid und reinigt über eine Kieselgelsäule mit Methylenchlorid/Methanol 99 : 1. Nach Anreiben mit Ligroin/Äther 1 : 1 und Absaugen werden 30 g 2-Benzyl-4-(2,3-epoxy-propoxy)-indazol vom Schmp. 66–68 °C erhalten (51% d. Th.).

Beispiel 30

4-<2-Hydroxy-3-[2-(3,4-dimethoxy-phenyl)-ethylamino]-propoxy>-indazol

19,1 g 2-Benzyl-4-<2-hydroxy-3-[2-(3,4-dimethoxy-phenyl)-ethylamino]-propoxy>-indazol (Herstellung siehe Beispiel 24) werden in 700 ml Methanol und 22,4 ml konzentrierte Salzsäure über 2 g 10%iger Palladium-Kohle hydriert. Nach Absaugen wird eingeengt, in Wasser gelöst, mit Natronlauge alkalisch gestellt und mit Methylenchlorid extrahiert. Die organische Phase wird eingeengt, mit Ether verrieben und abgesaugt.

Es werden 9,1 g Titelverbindung vom Schmp. 118–119 °C erhalten (59% d. Th.).

Durch Lösen in Ethanol und Zugabe von etherischer Salzsäure erhält man das Hydrochlorid vom Schmp. 157–159 °C.

In analoger Weise, wie oben beschrieben, erhält man:

| Bezeichnung | Ausb. % d. Th. | Smp. °C (Lösungsmittel) |
|---|---|---|
| a) 4-<2-Hydroxy-3-[2-(2-hydroxy-phenoxy)ethylamino]-propoxy>indazol aus 2-Benzyl-4-<2-hydroxy-3-[2-(2-benzyloxy-phenoxy)-ethylamino]propoxy>indazol (s. Bsp. 25) | 41 | 137–139 (Isopropanol) |
| b) 4-[2-Hydroxy-3-(2-phenoxy-ethylamino)propoxy]indazol aus 2-Benzyl-4-[2-hydroxy-3-(2-phenoxy-ethylamino)propoxy]indazol (s. Bsp. 26) | 88 | 134–135 (Essigester) |

| Bezeichnung | Ausb. % d. Th. | Smp. °C (Lösungsmittel) |
|---|---|---|
| c) 4-<2-Hydroxy-3-[2-(2-methoxy-phenoxy)ethylamino]-propoxy>indazol aus 2-Benzyl-4-<2-hydroxy-3-[2-(2-methoxy-phenoxy)-ethylamino]propoxy>indazol (s. Bsp. 27a) | 82 | 106–107 (Essigester) |
| d) 4-<2-Hydroxy-3-[2-(2-methoxy-phenoxy)propylamino]propoxy>indazol aus 2-Benzyl-4-<2-hydroxy-3-[2-(2-methoxy-phenoxy)propylamino]propoxy>indazol (s. Bsp. 27b) | 58 | 127–128 (Essigester) |
| e) 4-<2-Hydroxy-3-[2-(4-pyridyl)ethylamino]propoxy>-indazol aus 2-Benzyl-4-<2-hydroxy-3-[2-(4-pyridyl)ethylamino]-propoxy>indazol (s. Bsp. 27c) | 72 | 123–125 (Essigester) |
| f) 4-[2-Hydroxy-3-(benzo[b]-1,4-dioxan-2-yl-phenylamino)propoxy]indazol aus 2-Benzyl-4-[2-hydroxy-3-(benzo[b]-1,4-dioxan-2-yl-methylamino)propoxy]indazol (s. Bsp. 27d) | 54 | 142–143 (Essigester) |
| g) 4-<-Hydroxy-3-[2-(2-hydroxy-phenoxy)propylamino]-propoxy>indazol aus 2-Benzyl-4-<2-hydroxy-3-[2-(2-hydroxy-phenoxy)-N-benzyl-propylamino]propoxy>indazol (s. Bsp. 28) | 57 | 92–95 (Essigester) |
| h) 4-<2-Hydroxy-3-[2-(4-acetamido-phenoxy)ethylamino]propoxy>indazol aus 2-Benzyl-4-<2-hydroxy-3-[2-(4-acetamido-phenoxy)-N-benzyl-ethylamino]propoxy>indazol (s. Bsp. 29) | 49 | 130–133 (Ethanol/ Wasser) |
| i) 4-<2-Hydroxy-3-[2-(3,4-ethylendioxy-phenyl)ethylamino]propoxy>indazol aus 2-Benzyl-4-<2-hydroxy-3-[2-(3,4-ethylendioxy-phenyl)-ethylamino]propoxy>indazol (s. Bsp. 27e) | 59 | 125–126 (Essigester) |
| j) 4-<2-Hydroxy-3-[2-(3-ethoxy-4-methoxy-phenyl)-ethylamino]propoxy>indazol aus 2-Benzyl-4-<2-hydroxy-3-[2-(3-ethoxy-4-methoxy-phenyl)ethylamino]propoxy>indazol (s. Bsp. 27f) | 73 | 118–119 (Essigester) |
| k) 4-<2-Hydroxy-3-[2-(3,4,5-trimethoxy-phenyl)ethylamino]propoxy>indazol aus 2-Benzyl-4-<2-hydroxy-3-[2-(3,4,5-trimethoxy-phenyl)-ethylamino]propoxy>indazol (s. Bsp. 27g) | 63 | 171–172 (Essigester) |
| l) 4-<2-Hydroxy-3-[2-(4-hydroxy-3-methoxy-phenyl)-ethylamino]propoxy>indazol aus 2-Benzyl-4-<2-hydroxy-3-[2-(4-benzyloxy-3-methoxy-phenyl)ethylamino]propoxy>indazol (s. Bsp. 27h) | 57 | 139–141 (Isopropanol/ Essigester) |
| m) 4-<2-Hydroxy-3-[2-(4-butoxy-3-hydroxy-phenyl)ethylamino]propoxy>indazol aus 2-Benzyl-4-<2-hydroxy-3-[2-(3-benzyloxy-4-butoxy-phenyl)ethylamino]propoxy>indazol (s. Bsp. 27i) | 55 | 94–97 (Essigester) |
| n) 4-<2-Hydroxy-3-[2-(3,4-dihydroxy-phenyl)ethylamino]-propoxy>indazol-acetat aus 2-Benzyl-4-<2-hydroxy-3-[2-(3,4-dibenzyloxy-phenyl)-ethylamino]propoxy>indazol (s. Bsp. 27j) | 46 | 160–162 (Ethanol) |
| o) 4-<2-Hydroxy-3-[2-(2-pyridoxy)ethylamino]propoxy>-indazol aus 2-Benzyl-4-<2-hydroxy-3-[2-(2-pyridoxy)ethylamino]-propoxy>indazol (s. Bsp. 27k) | 52 | 113–115 (Essigester) |

Beispiel 31a

4-<2-Hydroxy-3-[1-(3,4-dimethoxy-phenyl)-propyl-2-amino]-propoxy>-indazol

Ein Gemisch aus 4 g 2-Benzyl-4-(3-amino-2-hydroxy-propoxy)-indazol und 3 g 3,4-Dimethoxy-phenyl-aceton wird in 100 ml Methanol in Gegenwart von 1,5 10%iger Palladiumkohle hydriert. Nach Aufnahme von 1 mol Wasserstoff werden 10 ml konzentrierter Salzsäure zugegeben und bis zur weiteren Aufnahme von 1 mol Wasserstoff geschüttelt.

Nach Absaugen und Einengen nimmt man in Wasser und Methylenchlorid auf. Nach Abtrennen der wässrigen Phase wird alkalisch gestellt und mit Methylenchlorid/Methanol 10 : 1 extrahiert. Nach Trocknen und Eindampfen wird das erhaltene Diastereomerengemisch aus Essigester umkristallisiert. Es werden so 1,1 g der Fraktion A der Titelverbindung vom Schmp. 110–112 °C erhalten (21% d. Th.).

Durch Einengen der Mutterlauge und Anreiben mit Ether werden nach Absaugen 1,5 g der Fraktion B der Titelverbindung vom Schmp. 66–73 °C erhalten (29% d. Th.).

Das als Ausgangsprodukt verwendete 2-Benzyl-4-(3-amino-2-hydroxy-propoxy)-indazol kann wie folgt erhalten werden:

12 g 2-Benzyl-4-(2,3-epoxy-propoxy)-indazol werden im Autoklav in 100 ml flüssigem Ammoniak 8 Stunden bei 50 °C geschüttelt. Man erhält nach Aufnehmen mit Essigester, Absaugen und Nachwaschen 9,3 g der Titelverbindung vom Schmp. 113–115 °C (73% d. Th.).

Beispiel 31b
4-<2-Hydroxy-3-[3-(3,4-dimethoxy-phenyl)-propylamino]-propoxy>-indazol-benzoat

6,6 g 4-(3-Amino-2-hydroxy-propoxy)-indazol (Rohprodukt) werden in 80 ml Methylenchlorid in Gegenwart von 3,1 ml Triethylamin mit 5,1 g 3-(3,4-Dimethoxy-phenyl)-propionylchlorid 48 Stunden bei Raumtemperatur gerührt. Nach Ausschütteln mit Wasser, Trocknen und Eindampfen verbleiben 9,1 g 4-<2-Hydroxy-3-[3-(3,4-dimethoxyphenyl) - propionamido] - propoxy > - indazol (Schmp. 148–150 °C aus Nitromethan).

Das erhaltene Rohprodukt wird in 120 ml wasserfreiem Tetrahydrofuran mit 1,7 g Lithiumaluminiumhydrid unter Stickstoffatmosphäre 26 Stunden bei 60 °C gerührt. Nach der üblichen Zerlegung und Aufarbeitung wird das erhaltene Öl über eine Kieselgelsäule (Laufmittel : Essigester-Methanol 9 : 1–6 : 4) gereinigt. Nach Einengen der gewünschten Fraktionen werden 1,7 g farbloses Öl erhalten. Durch Auflösen in Ethanol und Zugabe der berechneten Menge Benzoesäure werden nach Einengen und Zusatz von Ether 1,75 g 4-<2-Hydroxy-3-[3-(3,4-dimethoxy-phenyl)-propylamino]-propoxy>-indazol-benzoat vom Schmp. 131–134 °C (15% d. Th.) erhalten.

Das als Ausgangsprodukt verwendete 4-(3-Amino-2-hydroxy-propoxy)-indazol kann wie folgt erhalten werden:

5,1 g 1-Acetyl-4-(2,3-epoxy-propoxy)-indazol, gelöst in 50 ml Methanol werden im Autoklaven mit 250 ml flüssigem Ammoniak 8 Stunden bei 50 °C gerührt. Man behandelt die erhaltene Lösung mit Aktivkohle, engt ein und erhält 6,6 g der Titelverbindung als Rohprodukt, welches ohne weitere Reinigung zur nächsten Stufe eingesetzt werden kann.

Beispiel 32
1-Pivaloyl-4-<2-pivaloyloxy-3-[2-(3,4-dimethoxy-phenyl)-ethylamino]-propoxy>-indazol-hydrochlorid

Man rührt ein Gemisch aus 4,2 g 4-<2-Hydroxy-3-[2-(3,4-dimethoxy-phenyl)-äthylamino]-propoxy->-indazol-hydrochlorid (Herstellung s. Beispiel 30), 4,2 ml Pivalinsäureanhydrid und 35 ml Pivalinsäure 50–60 Stunden bei 75 °C. Die erhaltene feste Masse wird mit Ligroin gerührt, abgesaugt und mit Ligroin gewaschen. Es verbleiben 4,1 g Titelverbindung vom Schmp. 155–158 °C (69% d. Th.).

Beispiel 33
4-<2-Pivaloyloxy-3-[2-(3,4-dimethoxy-phenyl)-ethylamino]-propoxy>-indazol

4,0 g 1-Pivaloyl-4-<2-pivaloyloxy-3-[2-(3,4-dimethoxy-phenyl)-ethylamino]-propoxy>-indazol-hydrochlorid (Herstellung siehe Beispiel 32) werden mit 150 ml Isopropylamin 2,5 Stunden unter Rückfluss erhitzt. Man engt ein, löst in Ether, schüttelt mit 1n Natronlauge und reinigt die organische Phase chromatographisch über eine Kieselgelsäule (Laufmittel: Methylenchlorid/Essigester 1 : 1). Es werden 2,7 g Titelverbindung als Öl erhalten (85% d. Th.).

Beispiel 34
4-<2-Hydroxy-3-[2-(3,4-dimethoxy-phenyl)-ethylamino]-propoxy>-1-formylindolin-benzoat

2,2 g 4-(2,3-Hypoxy-propoxy)-1-formylindolin und 1,8 g 2-(3,4-Dimethoxy-phenyl)-ethylamin werden in 50 ml n-Butanol 18 Stunden bei Raumtemperatur gerührt, im Vakuum eingeengt und in Ether und 1n Milchsäure aufgenommen. Die wässrige Phase wird mit 2n Natronlauge alkalisch gestellt und mit Methylenchlorid extrahiert. Nach Trocknen und Eindampfen verbleiben 4 g Rohprodukt. Dieses wird über eine Kieselgelsäule mit Methylenchlorid/Methanol 95 : 5 gereinigt. So werden 1,3 g gereinigte Base erhalten, welche in 50 ml Essigester gelöst und mit der äquivalenten Menge Benzoesäure versetzt werden. Nach Absaugen verbleiben 1,3 g 4-<2-Hydroxy-3-[2-(3,4-dimethoxy-phenyl)-ethylamino]-propoxy>-1-formylindolin-benzoat vom Schmp. 136–138 °C (24% d. Th.).

Beispiel 35
In analoger Weise, wie in Beispiel 34 beschrieben, erhält man:

| Bezeichnung | Ausb. % d. Th. | Smp. °C (Lösungsmittel) |
|---|---|---|
| a) 4-<2-Hydroxy-3-[2-(4-fluor-phenoxy)ethylamino]propoxy>-1-formylindolin-benzoat aus 4-(2,3-Epoxy-propoxy)-1-formylindolin und 2-(4-Fluorphenoxy)ethylamin | 20 | 121–123 (Essigester) |

| Bezeichnung | Ausb. % d. Th. | Smp. °C (Lösungsmittel) |
|---|---|---|
| b) 4-<2-Hydroxy-3-[2-(2-methoxy-phenoxy)ethylamino]-propoxy>-1-formylindolin-benzoat aus 4-(2,3-Epoxy-propoxy)-1-formylindolin und 2-(2-Methoxy-phenoxy)ethylamin | 19 | 78–79 (Essigester) |
| c) 4-<2-Hydroxy-3-[2-(2-methoxy-phenoxy)-N-benzyl-propyl-amino]propoxy>-1-formylindolin aus 4-(2,3-Epoxy-propoxy)-1-formyl-indolin und 2-(2-Methoxy-phenoxy)-N-benzyl-propylamin | 90 | Öl |
| d) 4-<2-Hydroxy-3-[2-(2-benzyloxy-phenoxy)-N-benzyl-ethylamino]propoxy>-1-formylindol aus 4-(2,3-Epoxy-propoxy)-1-formylindolin und 2-(2-Benzyloxy-phenoxy)-N-benzyl-ethylamin | 95 | Öl |
| e) 4-[2-Hydroxy-3-(2-phenyl-ethylamino)propoxy]-1-formylindolin aus 4-(2,3-Epoxy-propoxy)-1-Formylindolin und 2-Phenyl-ethylamin | 18 | 121–123 (Essigester) |
| f) 4-<2-Hydroxy-3-[2-(2-chlor-phenoxy)ethylamino]-propoxy>-1-formylindolin aus 4-(2,3-Epoxy-propoxy)-1-formylindolin und 2-(2-Chlor-phenoxy)ethylamin | 15 | 104–107 (Essigester) |
| g) 4-<2-Hydroxy-3-[2-(2-methylmercapto-phenoxy)-ethylamino]propoxy>-1-formylindolin aus 4-(2,3-Epoxy-propoxy)-1-formylindolin und 2-(Methylmercapto-phenoxy)ethylamin | 20 | 129–130 (Methanol) |

Beispiel 36

4-<2-Hydroxy-3-[2-(2-methoxy-phenoxy)-propylamino]-propoxy>-1-formylindolin-benzoat

Man hydriert 6,7 g 4-<2-Hydroxy-3-[2-(2-methoxy-phenoxy)-N-benzyl-propylamino]-propoxy>-1-formylindolin (Herstellung siehe Beispiel 35c) in 250 ml Methanol und 20 ml Triethylamin bei Raumtemperatur und 1 bar Wasserstoffdruck über 2 g 10%iger Palladiumkohle, filtriert, engt ein und löst den verbleibenden Rückstand in 50 ml Essigester und versetzt mit der äquivalenten Menge Benzoesäure. Nach Absaugen erhält man 2,1 g 4-<2-Hydroxy-3-[2-(2-methoxy-phenoxy)-propylamino]-propoxy>-1-formylindolin-benzoat vom Schmp. 131–135 °C (31% d Th.).

Beispiel 37

In analoger Weise, wie in Beispiel 36 beschrieben, erhält man:

| Bezeichnung | Ausb. % d. Th. | Smp. °C (Lösungsmittel) |
|---|---|---|
| 4-<2-Hydroxy-3-[2-(2-hydroxy-phenoxy)ethylamino]propoxy> 1-formylindolin-acetat aus 4-<2-Hydroxy-3-[2-(2-benzyloxy-phenoxy)-N-benzyl-ethylamino]propoxy> 1-formylindolin (s. Bsp. 35d) | 41 | 171–173 (Essigester) |

Die zur Herstellung der vorstehenden Verbindungen benötigten Ausgangsstoffe werden wie folgt hergestellt:

4-(2,3-Epoxy-propoxy)-1-formylindolin

48,6 g 2-Benzyloxy-6-nitrotoluol gelöst in 670 ml Dimethylformamid werden mit 29,9 g Paraformaldehyd versetzt und anschliessend werden 200 ml 1n Kalium-tert.-butylatlösung zugetropft. Nach 1 Stunde Rühren bei Raumtemperatur wird in 3 l Eiswasser eingerührt und mit Ether extrahiert. Die Etherphase wird mit Natriumsulfat getrocknet und im Vakuum eingeengt. Es verbleiben 62 g 2-Benzyloxy-6-nitrophenylethanol, welches als Rohprodukt in die nächste Stufe eingesetzt wird.

62 g 2-Benzyloxy-6-nitro-phenylethanol werden in 500 ml wasserfreiem Pyridin gelöst und unter Kühlen bei ca. 10 °C mit 47,7 g p-Toluolsulfonylchlorid versetzt. Man lässt auf Raumtemperatur ansteigen und rührt bis zur völligen Umsetzung ca. 10 Stunden. Die Reaktionslösung wird in Eiswasser eingerührt. Nach Absaugen, Waschen mit Wasser und Trocknen verbleiben 74 g p-Toluolsulfonsäure-2-(2-benzyloxy-6-nitro-phenyl)-ethylester vom Schmp. 96–98 °C (86% d. Th., bezogen auf 2-Benzyloxy-6-nitrotoluol).

74 g p-Toluolsulfonsäure-2-(2-benzyloxy-6-nitro-phenyl)-ethylester werden in 2 l Ethylenglykolmonomethylether gelöst, mit 5 g 10%iger Palladium-Aktivkohle versetzt und bei Raumtemperatur und 1 bar Wasserstoffdruck hydriert. Nach Entfernen des Katalysators wird eingeengt und der Rückstand mit einer Mischung aus 227 ml Essigsäureanhydrid und 91 ml Ameisensäure [nach C.W. Huffmann, J. org. Chem. 23, 727 (1958)] formyliert. Nach der Umsetzung wird mit Eiswasser zersetzt und mit Essigester extrahiert. Die organische Phase wird neutralisiert, mit Natriumsulfat getrocknet und im Vakuum eingedampft. Der verbleibende Rückstand wird mit 320 ml Epichlorhydrin vermischt und mit 173 ml 2n Natriummethylatlösung versetzt. Nach Rühren über Nacht wird eingeengt und der Rückstand in Wasser und Essigester gelöst. Aus dem Essigesterrückstand werden durch Anreiben mit Isopropylalkohol und Absaugen 15,8 g 4-(2,3-Epoxy-propoxy)-1-formylindolin vom Schmp. 88–89 °C erhalten (42% d. Th.).

Beispiel 38

Es wurden Tabletten hergestellt: Jede Tablette enthält 10 mg 4-<2-Hydroxy-3-[2-(2-methoxy-phenoxy)-ethylamino]-propoxy>-methyl-2-benzimidazolinon-hydrochlorid. Die Tabletten wurden gemäss der folgenden Rezeptur hergestellt:

| | |
|---|---|
| 4-<2-Hydroxy-3-[2-(2-methoxy-phenoxy)-ethylamino]-propoxy>-methyl-2-benzimidazolinon-hydrochlorid | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpresst.

Beispiel 39

In analoger Weise, wie in Beispiel 23 beschrieben, erhält man:

| Bezeichnung | Ausb. % d. Th. | Fp. °C (Lösungsmittel) |
|---|---|---|
| a) 4-<2-Hydroxy-3-[2-(2-allylphenoxy)-ethyl-amino]-propoxy>-indazol aus 4-(2,3-Epoxypropoxy)-indazol und 2-(2-Allylphenoxy)-ethylamin | 47 | 125–126 (Essigester) |
| b) 4-<2-Hydroxy-3-[2-(2-allyloxyphenoxy)-ethyl-amino]-propoxy>-indazol aus 4-(2,3-Epoxypropoxy)-indazol und 2-(2-Allyloxyphenoxy)-ethylamin | 45 | 137–139 (Essigester) |

Versuchsprotokoll

Die erfindungsgemässen Verbindungen wurden hinsichtlich ihrer vasodilatierenden und β-blokkierenden Aktivität an Kaninchen geprüft. Da beide Eigenschaften nicht in einem einzigen Versuchsmodell geprüft werden können, mussten verschiedene Versuchsanordnungen gewählt werden.

a) Prüfung auf vasodilatierende Aktivität

Kaninchen wurden mit 26%igem Urethan 5 ml/kg narkotisiert. Zur fortlaufenden Messung des arteriellen Blutdruckes wurde ein Katheter in die A. femoralis implantiert. Die Messung des Blutdruckes erfolgte mittels eines elektromechanischen Druckwandlers (Statham P 23 Db). Die Impulse wurden auf einem Direktschreiber aufgezeichnet und nach Eichen mit einem Quecksilbermanometer ausgewertet.

Nach Ermittlung des Ausgangswertes wurden beide Halsschlagadern (A. carotis) für 2 Minuten occludiert und auf diese Weise der Blutdruck temporär erhöht (Carotis-Sinus-Entlastungs-Reflex – CSE). Anschliessend wurde die Prüfsubstanz in der niedrigsten Prüfdosis (0,125 mg/kg) i.v. injiziert und 8 Minuten später der CSE-Reflex erneut ausgelöst. Im Abstand von jeweils 15 Minuten wurden in logarithmisch steigender Dosierung (Faktor 2) die Prüfsubstanzen erneut injiziert und der CSE wiederum ausgelöst.

Substanzen, die unter diesen Bedingungen den Anstieg des Blutdruckes unter CSE abschwächen, können als vasodilatierend angesehen werden. Zur Abschätzung der blutdrucksenkenden Wirkqualität wurde von den Prüfsubstanzen die Dosis, die den CSE-Reflex um 30 mm Hg abschwächt ($ED_{-30\,mm\,Hg}$), berechnet.

b) Prüfung auf β-blockierende Aktivität

Kaninchen wurden in Holzkäfigen fixiert, die Herzfrequenz wurde bei ihnen über Stichelektroden abgeleitet und auf einem Frequenzzählgerät (Messzeit 15 Sekunden) abgelesen. Über eine Ohrvene wurde zunächst 1 µg/kg Isoprenalin i.v. injiziert, was eine Erhöhung der Herzfrequenz von ca. 210 Schlägen/Min. auf ca. 370 Schläge/Min. bewirkt. Anschliessend wurden die Prüfsubstanzen in steigender Dosierung (siehe Methode a) i.v. zugeführt und die Herzfrequenz nach Isoprenalin erneut abgelesen. Die Hemmung der Isoprenalin-Tachycardie ist als Mass für die β-Blokkade anzusehen. Es wurde die Dosis der Prüfsubstanzen bestimmt, die den Anstieg von Isoprenalin um 50% abschwächt ($HD_{50}$).

Die Daten aus beiden Versuchen sind in Tabelle I wiedergegeben. Die Berechnung der äquieffektiven Dosen ($ED_{-30\,mm\,Hg}$ bzw. $HD_{50}$) wurden auf logarithmischer Basis 4–6 bzw. 6–10 Einzelversuchen vorgenommen. Die aus beiden Versuchen errechneten Dosen ($HD_{50}$ und $ED_{-30\,mm\,Hg}$) wurden ins Verhältnis zueinander gesetzt.

Alle geprüften Substanzen besitzen starke β-blockierende Eigenschaften und darüber hinaus eine ausgeprägte blutdrucksenkende Qualität.

Da beide Wirkungen erwünscht sind, sollte das Auftreten beider Effekte durch möglichst naheliegende Dosen bewirkt werden, da sonst der Nutzen einer 2. Wirkqualität (wegen relativer Unterdosierung) nicht relevant würde. Optimal erscheint aus theoretischer Sicht ein Quotient von 1. Da jedoch klinische Erfahrungen noch nicht vorliegen, kommt auch allen Substanzen mit nicht allzu stark von 1 abweichenden Quotienten Interesse zu.

Tabelle I
Vasodilatierende und β-blockierende Aktivität der erfindungsgemässen Substanzen im Vergleich zu dem Handelsprodukt
PROPRANOLOL = 1-Isopropylamino-3-(1-naphthyloxy)-2-propanol

| Beispiel Nr. | $ED_{-30mmHg}$ (vasodilat. Akt.) (µg/kg i.v.) | $HD_{50}$ (β-blockierende Akt.) (µg/kg i.v.) | $\frac{HD_{50}}{ED_{-30mmHg}}$ |
|---|---|---|---|
| 2d | 641 | 406 | 0,63 |
| 3 | 1410 | 2485 | 1,76 |
| 4a | 3420 | 762 | 0,22 |
| 4b | 1910 | 3460 | 1,81 |
| 4c | 2240 | 5222 | 2,33 |
| 4f | 1660 | 931 | 0,51 |
| 4h | 3170 | 1338 | 0,42 |
| 4i | 5790 | 4338 | 0,75 |
| 7 | 3060 | 544 | 0,18 |
| 11 | 1160 | 233 | 0,20 |
| 15 | 400 | 59 | 0,15 |
| 16a | 1359 | 160 | 0,12 |
| 16b | 1580 | 487 | 0,31 |
| 16c | 1920 | 569 | 0,30 |
| 16l | 280 | 105 | 0,36 |
| 18c | 420 | 252 | 0,60 |
| 30f | 1980 | 154 | 0,21 |
| 34 | 3780 | 1253 | 0,33 |
| 35b | 1620 | 991 | 0,61 |
| Propranolol | >3550 | 393 | <0,11 |

**Patentansprüche**

1. Heterocyclische Oxypropanol-Derivate der allgemeinen Formel I

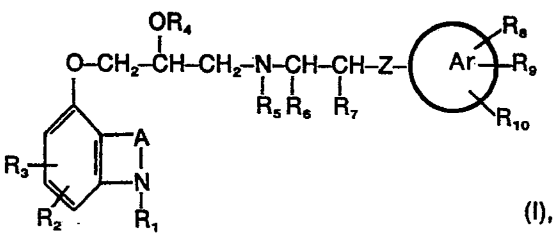

(I),

in welcher

$R_1$ Wasserstoff, eine Aralkyl-, $C_{1-6}$-Alkyl- oder $C_{1-8}$-Alkanoylgruppe,

$R_2$ und $R_3$, die gleich oder verschieden sein können, jeweils Wasserstoff, eine Alkoxycarbonyl-, $C_{1-6}$-Alkyl-, $C_{1-4}$-Hydroxyalkyl- oder ($C_{1-8}$-Alkanoyl)alkylgruppe sowie gemeinsam einen Alkylenrest mit bis 6 C-Atomen,

$R_4$ Wasserstoff, eine $C_{1-8}$-Alkanoylgruppe oder eine Aroylgruppe,

$R_5$ Wasserstoff, eine $C_{1-6}$-Alkylgruppe oder eine Aralkylgruppe,

$R_6$ Wasserstoff oder eine $C_{1-6}$-Alkylgruppe,

$R_7$ Wasserstoff, eine Hydroxygruppe oder eine $C_{1-6}$-Alkylgruppe,

Z einen Valenzstrich, eine Methylengruppe, ein Sauerstoff- oder Schwefelatom,

Ar einen carbocyclischen Arylrest oder einen Pyridylrest,

$R_8$, $R_9$ und $R_{10}$ die gleich oder verschieden sein können, jeweils Wasserstoff, Halogen, eine Hydroxygruppe, eine $C_{1-8}$-Alkanoylgruppe, eine $C_{1-6}$-Alkylgruppe, eine Alkenylgruppe mit bis 8 C-Atomen, eine $C_{1-6}$-Alkoxygruppe, eine Aralkoxygruppe, die Allyloxygruppe, eine $C_{1-6}$-Alkylthiogruppe, eine Aminocarbonylgruppe, eine Aminosulfonylgruppe, eine $C_{1-8}$-Alkanoylaminogruppe oder $R_8$ und $R_9$ gemeinsam eine Niederalkylendioxygruppe oder $R_8$ gemeinsam mit $R_7$ auch eine $-CH_2-O-$ Gruppe und

A die Gruppe $X_1$–$Y_1$, in der $X_1$ einen Methylen- oder –$NR_{11}$-Rest, wobei $R_{11}$ Wasserstoff oder eine $C_{1-6}$-Alkylgruppe bedeutet, und $Y_1$ einen Methylenrest oder die Gruppe >C=Q, wobei Q für Sauerstoff oder Schwefel steht, vorstellen, oder die Gruppe $X_2$=$Y_2$, in der $X_2$ und $Y_2$ gleich oder verschieden sein können und jeweils für ein Stickstoffatom oder für eine =$\overset{|}{C}R_{12}$–Gruppe stehen, wobei $R_{12}$ Wasserstoff, eine Alkoxycarbonylgruppe oder eine $C_{1-4}$-Alkylgruppe vorstellt, wobei für den Fall, dass $X_2Y_2$ eine –CH=N-Gruppe und $R_1$ eine Aralkylgruppe oder eine $C_{1-6}$-Alkylgruppe vorstellen, diese wegen der Tautomeriefähigkeit der Indazole auch an dem für $Y_2$ stehenden Stickstoffatom lokalisiert sein kann, mit der Massgabe, dass jeweils $Y_1$ mit dem Rest N–$R_1$ der allgemeinen Formel I verbunden ist, bedeuten, wobei für den Fall, dass Q ein Sauerstoffatom oder $X_2$=$Y_2$ eine –$CR_{12}$=$CR_{12}$-Gruppe bedeutet, entweder die beiden Reste $R_2$ und $R_3$ nicht gleichzeitig Wasserstoff sein können oder $R_7$ und $R_8$ zusammen eine –$CH_2$–O-Brücke bilden müssen, sowie deren pharmakologisch verträglichen Salze.

2. Verfahren zur Herstellung von Aminopropanolderivaten der allgemeinen Formel I

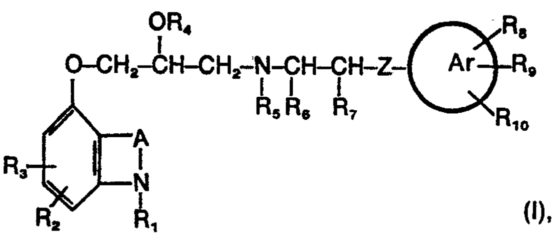

(I),

in welcher

$R_1$ Wasserstoff, eine Aralkyl-, $C_{1-4}$-Alkyl- oder $C_{1-8}$-Alkanoylgruppe,

$R_2$ und $R_3$, die gleich oder verschieden sein können, jeweils Wasserstoff, eine Alkoxycarbonyl-, $C_{1-6}$-Alkyl-, $C_{1-4}$-Hydroxyalkyl- oder ($C_{1-8}$-Alkanoyl)oxyalkylgruppe sowie gemeinsam einen Alkylenrest mit bis 6 C-Atomen,

$R_4$ Wasserstoff, eine $C_{1-8}$-Alkanoylgruppe oder eine Aroylgruppe,

$R_5$ Wasserstoff, eine $C_{1-6}$-Alkylgruppe oder eine Aralkylgruppe,

$R_6$ Wasserstoff oder eine $C_{1-6}$-Alkylgruppe,

$R_7$ Wasserstoff, eine Hydroxygruppe oder eine $C_{1-6}$-Alkylgruppe,

Z einen Valenzstrich, eine Methylengruppe, ein Sauerstoff- oder Schwefelatom,

Ar einen carbocyclischen Arylrest oder einen Pyridylrest,

$R_8$, $R_9$ und $R_{10}$, die gleich oder verschieden sein können, jeweils Wasserstoff, Halogen, eine Hydroxygruppe, eine $C_{1-8}$-Alkanoylgruppe, eine $C_{1-6}$-Alkylgruppe, eine Alkenylgruppe mit bis 6 C-Atomen, eine $C_{1-6}$-Alkoxygruppe, eine Aralkoxygruppe, die Allyloxygruppe, eine $C_{1-6}$-Alkylthiogruppe, eine Aminocarbonylgruppe, eine Aminosulfonylgruppe, eine $C_{1-8}$-Alkanoylaminogruppe oder $R_8$ und $R_9$ gemeinsam eine Niederalkylendioxygruppe oder $R_8$ gemeinsam mit $R_7$ auch eine –$CH_2$–O-Gruppe und

A die Gruppe $X_1$–$Y_1$, in der $X_1$ einen Methylen- oder –$NR_{11}$-Rest, wobei $R_{11}$ Wasserstoff oder eine $C_{1-6}$-Alkylgruppe bedeutet, und $Y_1$ einen Methylenrest oder die Gruppe >C=Q, wobei Q für Sauerstoff oder Schwefel steht, vorstellen, oder die Gruppe $X_2$=$Y_2$, in der $X_2$ und $Y_2$ gleich oder verschieden sein können und jeweils für ein Stickstoffatom oder für eine =$\overset{|}{C}R_{12}$-Gruppe stehen, wobei $R_{12}$ Wasserstoff, eine Alkoxycarbonyl- oder $C_{1-6}$-Alkylgruppe vorstellt, wobei für den Fall, dass $X_2$=$Y_2$ eine –CH=N-Gruppe und $R_1$ eine Aralkyl- oder $C_{1-6}$-Alkylgruppe vorstellen, diese wegen der Tautomeriefähigkeit der Indazole auch an dem für $Y_2$ stehenden Stickstoffatom lokalisiert sein kann, mit der Massgabe, dass jeweils $Y_1$ mit dem Rest N–$R_1$ der allgemeinen Formel I verbunden ist, bedeuten, wobei für den Fall, dass Q ein Sauerstoffatom oder $X_2$=$Y_2$ eine –$CR_{12}$=$CR_{12}$-Gruppe, entweder die beiden Reste $R_2$ und $R_3$ nicht gleichzeitig Wasserstoff sein können oder $R_7$ und $R_8$ zusammen eine –$CH_2$–O-Brücke bilden müssen, sowie deren pharmakologisch verträglichen Salze, dadurch gekennzeichnet, dass man in an sich bekannter Weise entweder

a) eine Verbndung der Formel II

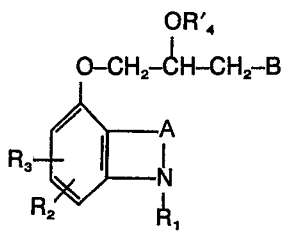

(II),

in welcher $R_1$, $R_2$, $R_3$ und A die oben genannte Bedeutung haben, B eine reaktive Gruppe darstellt und $R_{4p}$ die oben angegebene Bedeutung für $R_4$ hat oder zusammen mit B einen Valenzstrich bedeutet, mit einer Verbindung der Formel III

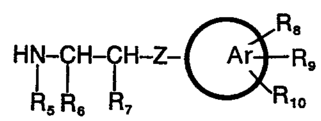

(III),

in welcher $R_5$, $R_6$, $R_7$, $R_8$, $R_9$, $R_{10}$, Z und Ar die oben angegebene Bedeutung haben, umsetzt oder

b) eine Verbindung der Formel IV

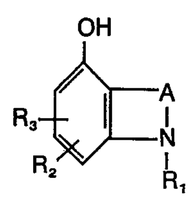

(IV),

in welcher $R_1$, $R_2$, $R_3$ und A die angegebene Bedeutung haben, mit einer Verbindung der Formel V

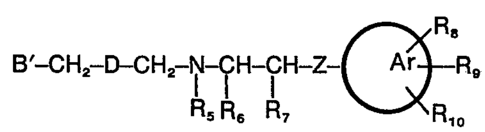

(V),

in welcher $R_6$, $R_7$, $R_8$, $R_9$, $R_{10}$, Z und Ar die oben angegebene Bedeutung haben, B′ eine reaktive Gruppe darstellt, D für eine >CO- oder >CH–$OR_4''$–Gruppe steht, wobei $R_4''$ die oben genannte Bedeutung für $R_4$ hat oder auch zusammen mit B′ einen Valenzstrich bedeutet und $R_5'$ die oben genannte Bedeutung für $R_5$ hat oder auch zusammen mit B′ eine Einfachbindung sein kann, umsetzt und, falls D die >CO-Gruppe bedeutet, anschliessend reduziert oder

c) eine Verbindung der Formel VI

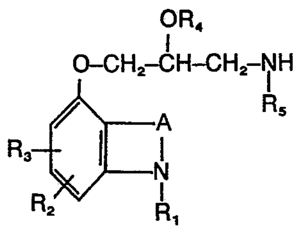

(VI),

in welcher $R_1$, $R_2$, $R_3$, $R_4$, $R_5$ und A die oben angegebene Bedeutung haben, mit einer Verbindung der Formel VII

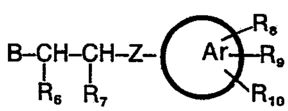

(VII),

in welcher B, $R_6$, $R_7$, $R_8$, $R_9$, $R_{10}$, Z und Ar die angegebene Bedeutung haben, umsetzt oder

d) ein Gemisch aus einer Verbindung der Formel VI mit einer Verbindung der Formel VIII

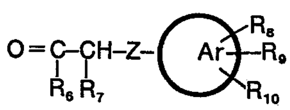

(VIII),

in welcher $R_6$, $R_7$, $R_8$, $R_9$, $R_{10}$, Z und Ar die angegebene Bedeutung haben, hydriert oder

e) eine Verbindung der Formel VI mit einer Verbindung der Formel IX

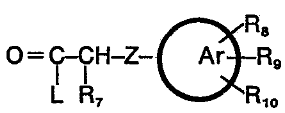

(IX),

in welcher $R_7$, $R_8$, $R_9$, $R_{10}$, Z und Ar die angegebene Bedeutung haben und L einen reaktiven Rest darstellt, umsetzt und das erhaltene Amid anschliessend reduziert oder

f) eine Verbindung der Formel X

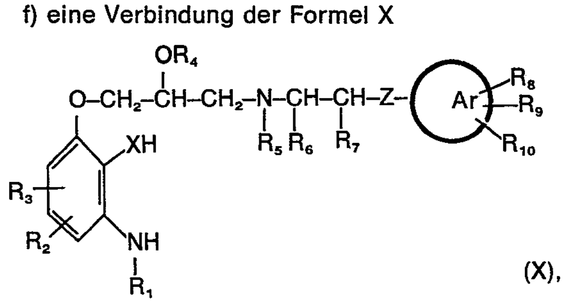

(X),

in welcher $R_1$, $R_2$, $R_3$, $R_4$, $R_5$, $R_6$, $R_7$, $R_8$, $R_9$, $R_{10}$, Z und Ar die angegebene Bedeutung haben und $XX_1$ oder $HX_2$ vorstellt, für den Fall, dass A die Gruppe $X_1$–$Y_1$ bedeutet, eine Verbindung, in der $X=X_1$ darstellt, mit einer Verbindung der allgemeinen Formel XIa

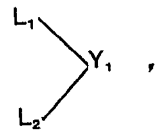

(XIa)

in der $Y_1$ die oben angegebene Bedeutung hat, $L_1$ ein Wasserstoffatom, die Hydroxygruppe oder einen reaktiven Rest T und $L_2$ ein Wasserstoffatom oder einen reaktiven Rest T bedeuten, oder für den Fall, dass A die Gruppe $X_2=Y_2$ bedeutet, eine Verbindung, in der $X = HX_2$ darstellt, mit einer Verbindung der allgemeinen Formel XIb

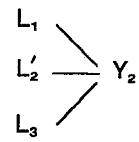

(XIb),

in der $L_1$ und $Y_2$ die oben angegebene Bedeutung haben, $L_2'$ ein Wasserstoffatom oder einen reaktiven Rest T und $L_3$ ein Wasserstoffatom oder zusammen mit $L_2'$ ein Sauerstoffatom darstellt, umsetzt und cyclisiert oder

g) eine Verbindung der Formel XII

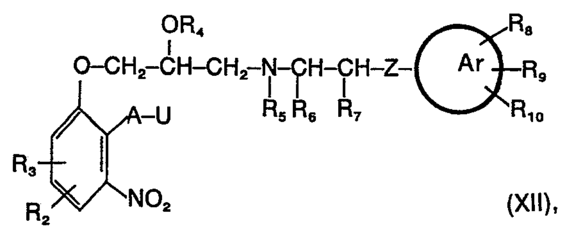

(XII),

in welcher $R_2$, $R_3$, $R_4$, $R_5$, $R_6$, $R_7$, $R_8$, $R_9$, $R_{10}$, Z, A und Ar die angegebene Bedeutung haben und U eine abspaltbare Gruppe darstellt, reduziert und cyclisiert und gegebenenfalls nachträglich in einer erhaltenen Verbindung der allgemeinen Formel I einen der Reste $R_1$, $R_2$, $R_3$, $R_4$, $R_5$, $R_8$, $R_9$, $R_{10}$ oder $R_{12}$ nach üblichen Methoden in einen anderen, durch den Anspruch definierten Rest $R_1$, $R_2$, $R_3$, $R_4$, $R_5$, $R_8$, $R_9$, $R_{10}$ oder $R_{12}$ überführt und die erhaltenen Verbindungen gewünschtenfalls in ihre pharmakologisch verträglichen Salze umwandelt.

3. Arzneimittel, enthaltend einen Wirkstoff der allgemeinen Formel I sowie an sich bekannte pharmakologisch verträgliche Träger- und Hilfsstoffe.

4. Aminopropanole gemäss Anspruch 1 oder deren Salze zur Verwendung als Arzneimittel zur Behandlung von Herz- und Kreislauferkrankungen.

## Claims

1. Heterocyclic oxypropanol derivatives of the general formula I

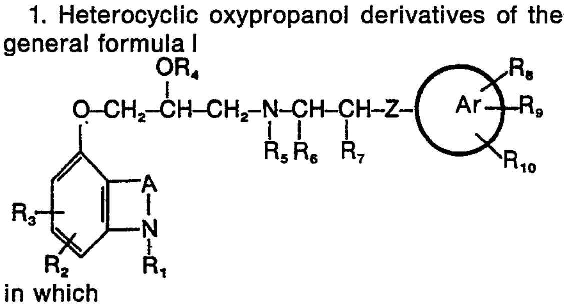

in which

$R_1$ signifies hydrogen, an aralkyl, $C_{1-6}$ alkyl or $C_{1-8}$ alkanoyl group,

$R_2$ and $R_3$, which can be the same or different, each signify hydrogen, an alkoxycarbonyl, $C_{1-6}$ alkyl, $C_{1-4}$ hydroxyalkyl or ($C_{1-8}$ alkanoyl)oxyalkyl group, as well as together signify an alkylene radical with up to 6 C-atoms,

$R_4$ signifies hydrogen, a $C_{1-8}$ alkanoyl group or an aroyl group,

$R_5$ signifies hydrogen, a $C_{1-6}$ alkyl group or an aralkyl group,

$R_6$ signifies hydrogen or a $C_{1-6}$ alkyl group,

$R_7$ signifies hydrogen, a hydroxyl group or a $C_{1-6}$ alkyl group,

Z signifies a valency bond, a methylene group, an oxygen or sulphur atom,

Ar signifies a carbocyclic aryl radical or a pyridyl radical,

$R_8$, $R_9$ and $R_{10}$, which can be the same or different, each signify hydrogen, halogen, a hydroxy group, a $C_{1-8}$ alkanoyl group, a $C_{1-8}$ alkyl group, an alkenyl group with up to 8 C-atoms, a $C_{1-6}$ alkoxy group, an aralkoxy group, the allyloxy group, a $C_{1-6}$ alkylthio group, an aminocarbonyl group, an aminosulphonyl group, a $C_{1-8}$ alkanoylamino group or $R_8$ and $R_9$ together signify a lower alkylenedioxy group or $R_8$ together with $R_7$ also a $-CH_2-O-$ group and

A signifies the group $X_1-Y_1$, in which $X_1$ represents a methylene or $-NR_{11}$ radical, whereby $R_{11}$ signifies hydrogen or a $C_{1-6}$ alkyl group, and $Y_1$ represents a methylene radical or the group $>C=Q$, whereby Q stands for oxygen or sulphur; or the group $X_2=Y_2$, in which $X_2$ and $Y_2$ can be the same or different and each stands for a nitrogen atom or for a $=\overset{|}{C}R_{12}$ group, whereby $R_{12}$ represents hydrogen, an alkoxycarbonyl group or a $C_{1-4}$ alkyl group, whereby for the case in which $X_2=Y_2$ represents a $-CH=N-$ group and $R_1$ an aralkyl group or a $C_{1-6}$ alkyl group, because of the ability to tautomerise of the indoles, this can also be localised on the nitrogen atom standing for $Y_2$, with the proviso that in each case $Y_1$ is connected to the radical $N-R_1$ of the general formula I, whereby for the case in which Q signifies an oxygen atom or $X_2=Y_2$ a $-CR_{12}=CR_{12}-$ group, either the two residues $R_2$ and $R_3$ cannot simultaneously be hydrogen or $R_7$ and $R_8$ must together form a $-CH_2-O-$ bridge, as well as their pharmacologically compatible salts.

2. Process for the preparation of aminopropanol derivatives of the general formula I

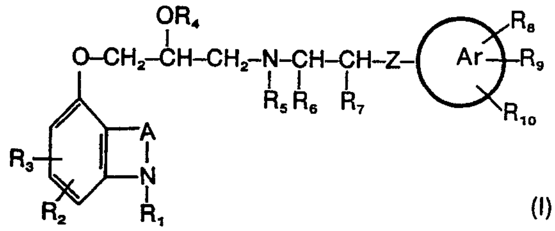

(I)

in which $R_1$ signifies hydrogen, an aralkyl, $C_{1-4}$ alkyl or $C_{1-8}$ alkanoyl group, $R_2$ and $R_3$, which can be the same or different, each signify hydrogen, an alkoxycarbonyl, $C_{1-6}$ alkyl, $C_{1-4}$ hydroxyalkyl or ($C_{1-8}$ alkanoyl)-oxyalkyl group, as well as together signify an alkylene radical with up to 6 C-atoms, $R_4$ signifies hydrogen, a $C_{1-6}$ alkanoyl group or an aroyl group, $R_5$ signifies hydrogen, a $C_{1-6}$ alkyl group or an aralkyl group, $R_6$ signifies hydrogen or a $C_{1-6}$ alkyl group, $R_7$ signifies hydrogen, a hydroxyl group or a $C_{1-6}$ alkyl group, Z signifies a valency bond, a methylene group, an oxygen or sulphur atom, Ar signifies a carbocyclic aryl radical or a pyridyl radical, $R_8$, $R_9$ and $R_{10}$, which can be the same or different, each signify hydrogen, halogen, a hydroxyl group, a $C_{1-8}$ alkanoyl group, a $C_{1-6}$ alkyl group, an alkenyl group with up to 6 C-atoms, a $C_{1-6}$ alkoxy group, an aralkoxy group, the allyloxy group, a $C_{1-6}$ alkylthio group, an aminocarbonyl group, an aminosulphonyl group, a $C_{1-8}$ alkanoylamino group or $R_8$ and $R_9$ together signify a lower alkylenedioxy group or $R_8$ together with $R_7$ also signifies a $-CH_2-O-$ group and A signifies the group $X_1-Y_1$, in which $X_1$ signifies a methylene or $-NR_{11}$ radical, whereby $R_{11}$ signifies hydrogen or a $C_{1-6}$ alkyl group and $Y_1$ signifies a methylene radical or the group $>C=Q$, whereby Q stands for oxygen or sulphur; or the group $X_2=Y_2$, in which $X_2$ and $Y_2$ can be the same or different and each stands for a nitrogen atom or for a $=\overset{|}{C}R_{12}$ group, whereby $R_{12}$ represents hydrogen, an alkoxycarbonyl or $C_{1-6}$ alkyl group, whereby for the case in which $X_2=Y_2$ represents a $-CH=N-$ group and $R_1$ an aralkyl or $C_{1-6}$ alkyl group, this, because of the ability to tautomerise of the indazoles, can also be localised on the nitrogen atom standing for $Y_2$, with the proviso that in each case $Y_1$ is connected with the radical $N-R_1$ of the general formula I, whereby for the case in which Q signifies an oxygen atom or $X_2=Y_2$ a $-CR_{12}=CR_{12}-$ group, either the two residues $R_2$ and $R_3$ cannot simultaneously be hydrogen or $R_7$ and $R_8$ must together form a $-CH_2-O-$ bridge, as well as of their pharmacoligically compatible salts, characterised in that, in per se known manner, one either

a) reacts a compound of the formula II

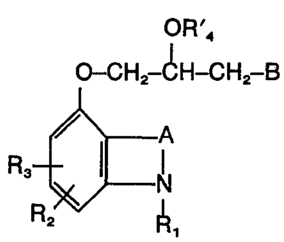

(II)

in which $R_1$, $R_2$, $R_3$ and A have the above-mentioned meaning, B represents a reactive group and $R_4'$ has the above-given meaning for $R_4$ or, together with B, signifies a valency bond, with a compound of the formula III

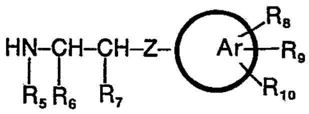 (III)

in which $R_5$, $R_6$, $R_7$, $R_8$, $R_9$, $R_{10}$, Z and Ar have the above-given meaning; or

b) reacts a compound of the formula IV

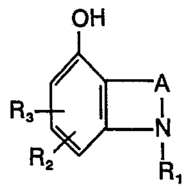 (IV),

in which $R_1$, $R_2$, $R_3$ and A have the given meaning, with a compound of the formula V

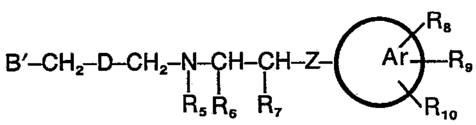 (V)

in which $R_6$, $R_7$, $R_8$, $R_9$, $R_{10}$, Z and Ar have the above-given meaning, B′ represents a reactive group, D stands for a >CO or >CH–$OR_4''$ group, whereby $R_4''$ has the above-mentioned meaning for $R_4$ or, together with B′, also signifies a valency bond and $R_5'$ has the above-mentioned meaning for $R_5$ or, together with B′, can also be a single bond, and, if D signifies the >CO group, subsequently reduces; or

c) reacts a compound of the formula VI

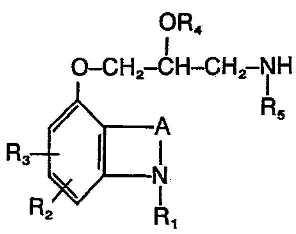 (VI),

in which $R_1$, $R_2$, $R_3$, $R_4$, $R_5$ and A have the above-given meaning, with a compound of the formula VII

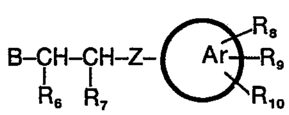 (VII),

in which B, $R_6$, $R_7$, $R_8$, $R_9$, $R_{10}$, Z and Ar have the given meaning; or

d) hydrogenates a mixture of a compound of the formula VI with a compound of the formula VIII

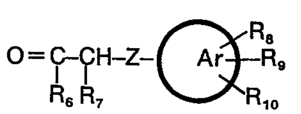 (VIII),

in which $R_6$, $R_7$, $R_8$, $R_9$, $R_{10}$, Z and Ar have the given meaning; or

e) reacts a compound of the formula VI with a compound of the formula IX

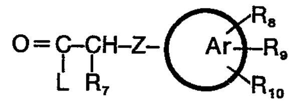 (IX),

in which $R_7$, $R_8$, $R_9$, $R_{10}$, Z and Ar have the given meaning and L represents a reactive residue, and subsequently reduces the amide obtained; or

f) a compound of the formula X

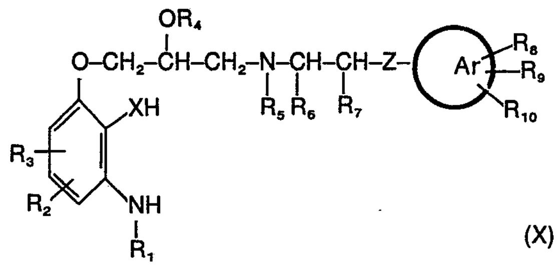 (X)

in which $R_1$, $R_2$, $R_3$, $R_4$, $R_5$, $R_6$, $R_7$, $R_8$, $R_9$, $R_{10}$, Z and Ar have the given meaning and X represents $X_1$ or $HX_2$, for the case in which A signifies the group $X_1$–$Y_1$, reacts and cyclises a compound in which $X = X_1$ with a compound of the general formula XIa

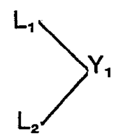 (XIa)

in which $Y_1$ has the above-given meaning, $L_1$ signifies a hydrogen atom, the hydroxyl group or a reactive residue T and $L_2$ a hydrogen atom or a reactive residue T, or for the case in which A signifies the group $X_2{=}Y_2$, reacts and cyclises a compound in which X represents $HX_2$ with a compound of the general formula XIb

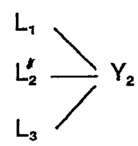 (XIb),

in which $L_1$ and $Y_2$ have the above-given meaning, $L_2'$ represents a hydrogen atom or a reactive residue T and $L_3$ a hydrogen atom or, together with $L_2'$, an oxygen atom; or

g) reduces and cyclises a compound of the formula XII

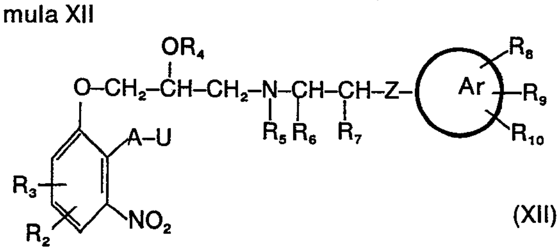 (XII)

in which $R_2$, $R_3$, $R_4$, $R_5$, $R_6$, $R_7$, $R_8$, $R_9$, $R_{10}$, Z, A and Ar have the given meaning and U represents a group which can be split off, and possibly subsequently, in a compound obtained of the general formula I, converts one of the residues $R_1$, $R_2$, $R_3$, $R_4$, $R_5$, $R_8$, $R_9$, $R_{10}$ or $R_{12}$ according to conventional methods into another residue $R_1$, $R_2$, $R_3$, $R_4$, $R_5$, $R_8$, $R_9$, $R_{10}$ or $R_{12}$ defined in the claim and, if desired, converts the compounds obtained into their pharmacologically compatible salts.

3. Medicaments containing an active material of the general formula I, as well as per se known pharmacologicslly compatible carrier and adjuvant materials.

4. Aminopropanols according to claim 1 or their salts for use as medicaments for the treatment of heart and circulatory diseases.

**Revendications**

1. Dérivés hétérocycliques d'oxypropanol de formule générale I

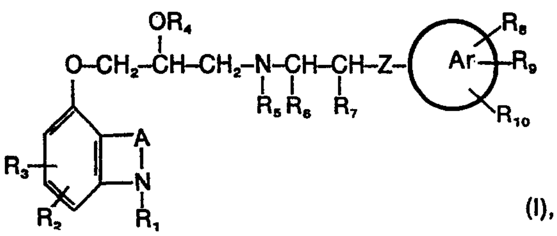

(I),

dans laquelle:

$R_1$ est de l'hydrogène, un groupe aralkyle, alkyle $C_{1-6}$, ou alcanoyle $C_{1-8}$,

$R_2$ et $R_3$ pouvant être identiques ou différents, sont chacun un atome d'hydrogène, un groupe alcoxycarbonyle, alkyle $C_{1-6}$, hydroxyalkyle $C_{1-4}$ ou (alcanoyl $C_{1-8}$) oxyalkyle, ou forment ensemble un reste alkylène ayant jusqu'à 6 atomes de carbone,

$R_4$ est de l'hydrogène, un groupe alcanoyle $C_{1-8}$ ou un groupe aroyle,

$R_5$ est de l'hydrogène, un groupe alkyle $C_{1-6}$ ou un groupe aralkyle,

$R_6$ est de l'hydrogène ou un groupe alkyle $C_{1-6}$,

$R_7$ est de l'hydrogène, un groupe hydroxyle ou un groupe alkyle $C_{1-6}$,

Z est un trait de valence, un groupe méthylène, un atome d'oxygène ou de soufre,

Ar est un reste aryle carbocyclique ou un reste pyridyle,

$R_8$, $R_9$ et $R_{10}$ pouvant être identiques ou différents, sont chacun de l'hydrogène, de l'halogène, un groupe hydroxyle, un groupe alcanoyle $C_{1-8}$, un groupe alkyle $C_{1-6}$, un groupe alcényle ayant jusqu'à 8 atomes de carbone, un groupe alcoxy $C_{1-6}$, un groupe aralcoxy, le groupe allyloxy, un groupe alkyle-thio $C_{1-6}$, un groupe aminocarbonyle, un groupe amino sulfonyle, un groupe alcanoylamino $C_{1-8}$, ou $R_8$ et $R_9$ forment ensemble un groupe alkylènedioxy inférieur ou bien $R_8$ forme avec $R_7$ aussi un groupe $-CH_2O-$, et

A est le groupe $X_1-Y_1$, dans lequel $X_1$ est un reste méthylène ou un reste $-NR_{11}$, où $R_{11}$ est de l'hydrogène ou un groupe alkyle $C_{1-6}$ et $Y_1$ un reste méthylène ou le groupe $>C=Q$, où Q désigne l'oxygène ou le soufre, ou, est le groupe $X_2=Y_2$, dans lequel $X_2$ et $Y_2$ sont identiques ou différents et désignent chacun un atome d'azote ou un groupe $=\overset{|}{C}R_{12}$, où $R_{12}$ est de l'hydrogène, un groupe alcoxycarbonyle ou un groupe alkyle $C_{1-4}$, et au cas où $X_2=Y_2$ est un groupe $-CH=N$ et $R_1$, un groupe aralkyle ou un groupe alkyle $C_{1-6}$, celui-ci peut être lié à l'atome d'azote représenté par $Y_2$, en raison des tautomères que les indazoles sont susceptibles de former, sous la condition que dans chaque cas $Y_1$ est lié au reste $N-R_1$ de la formule générale I, et dans le cas où Q est un atome d'oxygène ou $X_2=Y_2$, un groupe $-CR_{12}=CR_{12}$, ou bien les deux restes $R_2$ et $R_3$ ne sont pas en même temps de l'hydrogène, ou bien $R_7$ et $R_8$ doivent former ensemble un pont $-CH_2O-$, ainsi que leurs sels pharmacologiquement tolérables.

2. Procédé pour la préparation de dérivés d'aminopropanol de formule générale I

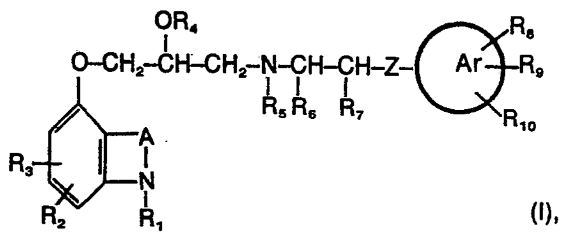

(I),

dans laquelle:

$R_1$ est de l'hydrogène, un groupe aralkyle, alkyle $C_{1-6}$, ou alcanoyle $C_{1-8}$,

$R_2$ et $R_3$ pouvant être identiques ou différents, sont chacun un atome d'hydrogène, un groupe alcoxycarbonyle, alkyle $C_{1-6}$, hydroxyalkyle $C_{1-4}$ ou (alcanoyl $C_{1-8}$)oxyalkyle, ou forment ensemble un reste alkylène ayant jusqu'à 6 atomes de carbone,

$R_4$ est de l'hydrogène, un groupe alcanoyle $C_{1-8}$ ou un groupe aroyle,

$R_5$ est de l'hydrogène, un groupe alkyle $C_{1-6}$ ou un groupe aralkyle,

$R_6$ est de l'hydrogène ou un groupe alkyle $C_{1-6}$,

$R_7$ est de l'hydrogène, un groupe hydroxyle ou un groupe alkyle $C_{1-6}$,

Z est un trait de valence, un groupe méthylène, un atome d'oxygène ou de soufre,

Ar est un reste aryle carbocyclique ou un reste pyridyle,

$R_8$, $R_9$ et $R_{10}$ pouvant être identiques ou différents, sont chacun de l'hydrogène, de l'halogène, un groupe hydroxyle, un groupe alcanoyle $C_{1-8}$, un groupe alkyle $C_{1-6}$, un groupe alcényle ayant jusqu'à 8 atomes de carbone, un groupe alcoxy $C_{1-8}$, un groupe aralcoxy, le groupe allyloxy, un groupe alkyle-thio $C_{1-6}$, un groupe aminocarbonyle, un groupe amino sulfonyle, un groupe alcanoylamino $C_{1-8}$, ou $R_8$ et $R_9$ forment ensemble un groupe alkylènedioxy inférieur ou bien $R_8$ forme avec $R_7$ aussi un groupe $-CH_2O-$,

A est le group $X_1-Y_1$, dans lequel $X_1$ est un reste méthylène ou un reste $-NR_{11}$, où $R_{11}$ est de l'hydrogène ou un groupe alkyle $C_{1-6}$ et $Y_1$ un reste méthylène ou le groupe $>C=Q$, où Q désigne l'oxygène ou le soufre, ou, est le groupe $X_2=Y_2$, dans lequel $X_2$ et $Y_2$ sont identiques ou différents et désignent chacun un atome d'azote ou un groupe $=\overset{|}{C}R_{12}$, où $R_{12}$ est de l'hydrogène, un groupe alcoxycarbonyle ou un groupe alkyle $C_{1-4}$, et au cas où $X_2=Y_2$ est un groupe $-CH=N$ et $R_1$, un groupe aralkyle ou un groupe alkyle $C_{1-6}$, celui-ci peut être lié à l'atome d'azote représenté par $Y_2$, en raison des tautomères que les indazoles sont susceptibles de former, sous la condition que dans chaque cas $Y_1$ est lié au reste $N-R_1$ de la formule générale I, et dans le cas où Q est un atome d'oxygène ou $X_2=Y_2$, un groupe $-CR_{12}=CR_{12}$, ou bien les deux restes $R_2$ et $R_3$ ne sont pas en même temps de l'hydrogène, ou bien $R_7$ et $R_8$ doivent former ensemble un pont $-CH_2O-$, ainsi que leurs sels pharmacologiquement tolérables, caractérisé en ce que de façon en soi connue, soit:

a) on fait réagir un composé de formule II

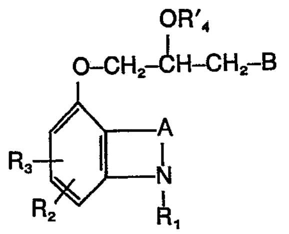

(II),

dans laquelle $R_1$, $R_2$, $R_3$ et A ont la signification donnée ci-dessus, B est un groupe réactif et $R_4'$ a la signification donnée ci-dessus pour $R_4$, ou forme ensemble avec B un trait de valence, avec un composé de formule III

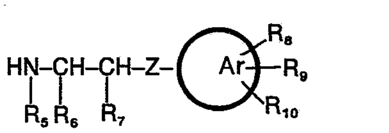

(III),

dans laquelle $R_5$, $R_6$, $R_7$, $R_8$, $R_9$, $R_{10}$, Z et Ar ont la signification ci-dessus, soit,

b) on fait réagir un composé de formule IV

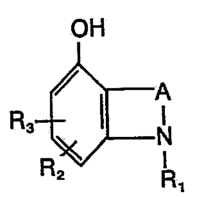

(IV),

dans laquelle $R_1$, $R_2$, $R_3$ et A sont la signification indiquée, avec un composé de formule V

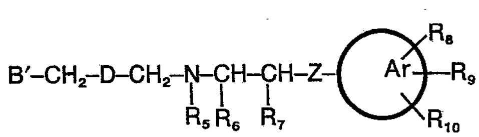

(V),

dans laquelle $R_6$, $R_7$, $R_8$, $R_9$, $R_{10}$, Z et Ar ont la signification indiquée ci-dessus, B' est un groupe réactif, D est un groupe >CO ou >CH–OR$_4''$ où $R_4''$ a la signification donnée ci-dessus pour $R_4$, ou forme ensemble avec B' un trait de valence et $R_5'$ a la signification donnée ci-dessus pour $R_5$, ou peut former avec B' une liaison simple et au cas où D est le groupe >CO on réduit ensuite, soit

c) on fait réagir un composé de formule VI

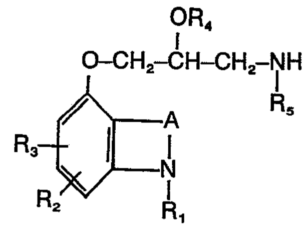

(VI),

dans laquelle $R_1$, $R_2$, $R_3$, $R_4$, $R_5$ et A ont la signification indiquée ci-dessus, avec un composé de formule VII

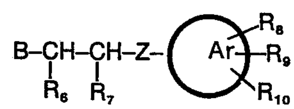

(VII),

dans laquelle B, $R_6$, $R_7$, $R_8$, $R_9$, $R_{10}$, Z et Ar ont la signification indiquée, soit

d) on soumet un mélange d'un composé de formule VI avec un composé de formule VIII

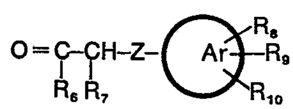

(VIII),

dans laquelle $R_6$, $R_7$, $R_8$, $R_9$, $R_{10}$, Z et Ar ont la signification indiquée, à une hydrogénation, soit

e) on fait réagir un composé de formule VI avec un composé de formule IX

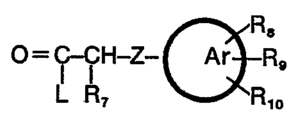

(IX),

dans laquelle $R_7$, $R_8$, $R_9$, $R_{10}$, Z et Ar ont la signification indiquée et L est un reste réactif et on réduit ensuite l'amide obtenu, soit

f) on fait réagir et on cyclise un composé de formule X

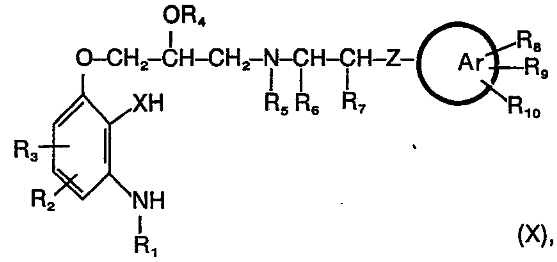

(X),

dans laquelle $R_1$, $R_2$, $R_3$, $R_4$, $R_5$, $R_6$, $R_7$, $R_8$, $R_9$, $R_{10}$, Z et Ar ont la signification indiquée et X représente $X_1$ ou $HX_2$, c'est-à-dire dans le cas où A est le groupe $X_1-Y_1$, un composé dans lequel $X = X_1$, avec un composé de formule générale XIa

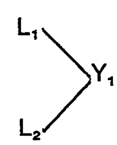

(XIa)

dans laquelle $Y_1$ a la signification indiquée ci-dessus, $L_1$ est un atome d'hydrogène, le groupe hydroxyle ou un reste réactif T et $L_2$ un atome d'hydrogène ou un reste T, ou dans le cas où A est le groupe $X_2=Y_2$, un composé dans lequel $X = HX_2$, avec un composé de formule générale XIb

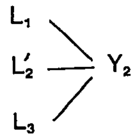

(XIb),

dans laquelle $L_1$ et $Y_2$ ont la signification donnée ci-dessus, $L_2'$ est un atome d'hydrogène ou un reste réactif T et $L_3$ est un atome d'hydrogène, ou forme ensemble avec $L_2'$ un atome d'oxygène, soit

g) on réduit et on cyclise un compose de formule XII

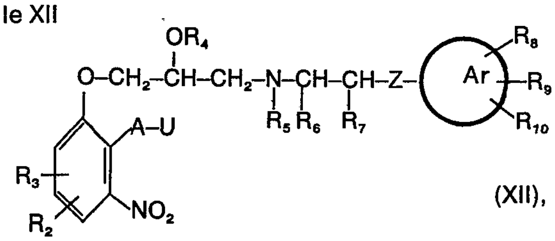

(XII),

dans laquelle $R_2$, $R_3$, $R_4$, $R_5$, $R_6$, $R_7$, $R_8$, $R_9$, $R_{10}$, Z, A et Ar ont la signification indiquée et U est un groupe éliminable, et éventuellement on transforme ultérieurement dans un composé de formule générale I obtenu, un des restes $R_1$, $R_2$, $R_3$, $R_4$, $R_5$, $R_8$, $R_9$, $R_{10}$ ou $R_{12}$ selon une méthode habituelle en un autre reste $R_1$, $R_2$, $R_3$, $R_4$, $R_5$, $R_8$, $R_9$, $R_{10}$ ou $R_{12}$ défini dans la revendication et on transforme les composés obtenus éventuellement en leurs sels pharmacologiquement tolérables.

3. Médicaments contenant une substance active de formule générale I ainsi que des substances de support et auxiliaires pharmacologiquement tolérables et en soi connues.

4. Des aminopropanols selon la revendication 1 ou leurs sels destinés à être employés comme médicaments pour le traitement des maladies du cœur et de la circulation.